# EUROPEAN PATENT SPECIFICATION

(11) **EP 0 831 887 B1**
(45) Date of publication and mention of the grant of the patent: **27.02.2002**
(21) Application number: 96916957.2
(22) Date of filing: 03.06.1996
(51) Int. Cl.: A61K 38/19

(54) **THERAPY USING LYMPHOTOXIN AND THROMBOPOIETIN**
THERAPIE UNTER VERWENDUNG VON LYMPHOTOXIN UND THROMBOPOIETIN
THERAPEUTIQUE EMPLOYANT LA LYMPHOTOXINE ET LA THROMBOPOIETINE

(30) Priority: 06.06.1995 US 470776
(43) Date of publication of application: 01.04.1998
(73) Proprietor: Genentech, Inc., South San Francisco, CA 94080-4990 (US)
(72) Inventor: WONG, Grace, H., W., Brookline-Chestnut Hill, MA 02167 (US)
(74) Representative: Kiddle, Simon John
(86) International application number: US9608343
(87) International publication number: WO9639171

(56) References cited:
- WO-A-96/01121
- Dialog Information Services, File 351, Dialog accession no. 010332326, WPI accession no. 95-234018/31, Genentech Inc: "Thrombopoietin polypeptide, ligand for mpl cytokine receptor-useful for treating thrombocytopenia and related diseases"; & LU,A,88573, 950601, 9532 See page 3, lines 15-19; page 15, lines 3-7; page 25, lines 24-25; page 94, lines 19-37; claims 36-38 in WO, A1, 9518858
- Dialog Information Services, file 155, Medline, Dialog accession no. 09392476, Medline accession no. 95322476, Wong GH et al: "Protective roles of cytokines against radiation: induction of mitochondrial MnSOD", Biochim Biophys Acta (NETHERLANDS) May 24 1995, 1271(1) p205-9
- Dialog Information Services, file 155, Medline, Dialog accession no. 09257094, Medline accession no. 95187094, Lok S. et al: "The structure, biology and potential therapeutic applications of recombi- nant thrombopoietin", Stem Cells (Dayt) (UNITED STATES) Nov 1994, 12 (6) p586-98

## Description

### Field of the Invention

The object of the invention is defined in the claims. The invention also relates to the use of lymphotoxin and thrombopoietin to treat hematopoietic conditions which can result from certain diseases or anti-cancer therapies.

### Background of the Invention

Tumor necrosis factor ("TNF") was first identified as a serum-derived factor which was cytotoxic for several transformed cell lines *in vitro* and caused necrosis of certain tumors *in* vivo. A similar factor was identified and referred to as lymphotoxin ("LT"). Due to observed similarities between TNF and LT in the early 1980's, it was proposed that TNF and LT be referred to as TNF-α and TNF-β. respectively. Scientific literature thus makes reference to both nomenclatures. As used in the present application, the term "TNF" refers to tumor necrosis factor or TNF-α, and the term "LT" refers to lymphotoxin or TNF-β.

Cloning of the TNF and LT proteins and further characterization of their respective biological activities revealed that the proteins differ in many aspects. [Aggarwal et al., Cytokines and Lipocortins in Inflammation and Differentiation, Wiley-Liss, Inc. 1990, pp. 375-384]. For instance. LT is a secreted, soluble protein of approximately 20 kDa (25 kDa if N- and O-glycosylated). TNF, in contrast, has no site for glycosylation and is synthesized with an apparent transmembrane domain that results in the original protein transcript being cell associated. Proteolysis of the cell associated TNF protein results in the release of the soluble form of the protein having a molecular weight of approximately 19 kDa. TNF is produced primarily by activated macrophages whereas LT is produced by activated lymphocytes. [Wong et al., Tumor Necrosis Factors: The Molecules and their Emerging Role in Medicine, Beutler, B., ed., Raven Press (1991), pp. 473-484]. The sequences encoding TNF and LT also differ. TNF and LT share only approximately 32% amino acid sequence identity. Regarding the different biological activities of TNF and LT, TNF increases production of.endothelial cell interleukin-1 ("IL-1") whereas LT has little effect. Further, TNF induces production of macrophage colony stimulating factor from macrophages whereas LT has no effect. These and other biological activities are discussed in Aggarwal, Tumor Necrosis Factors: Structure. Function and Mechanism of Action, Aggarwal and Vicek, eds. (1992), pp. 61-78.

TNF and LT are described further in the review articles by Spriggs, 'Tumor Necrosis Factor: Basic Principles and Preclinical Studies," Biologic Therapy of Cancer, DeVita et al., eds., J.B. Lippincott Company (1991) Ch. 16, pp. 354-377; Ruddle, Current Opinion in Immunology, 4:327-332 (1992); Wong et al., "Tumor Necrosis Factor," Human Monocytes, Academic Press (1989), pp. 195-215; and Paul et al., Ann. Rev. Immunol., 6:407-438 (1988).

Although increased numbers of cancer treatments have become available in recent years, a need exists for improved types of cancer therapy and therapy protocols. Some chemotherapies and radiation therapies show relatively promising results but are often limited in terms of dosages which can be tolerated by patients. Further, cancer treatments are often accompanied by adverse side effects, such as destruction of normal or healthy tissues and cells, impairment of the lymphoid and hematopoietic systems, or hair loss (also referred to as alopecia).

As discussed above, TNF has been reported to have certain anti-cancer activity. [see, e.g., Haranaka et al., Int. J. Cancer, 34:263-267 (1984); Nishiguchi et al., Int. J. Radiation Oncology, 18:555-558 (1990)]. In particular, the effects of TNF in certain cancer treatments have been reported. TNF, for example, has been described as having certain protective effects against radiation under particular experimental conditions. U.S. Patent No. 4,861,587, issued August 29, 1989 to Urbaschek et al. discloses the use of TNF in the treatment of radiation damage. The effect of TNF in protecting bone marrow precursor cells from irradiation has also been described. [See, e.g., Dalmau et al., Bone Marrow Transplantation, 12:551-563 (1993); Wong et al., Science, 242:941-944 (1988); Neta et al., J. Immunol., 140:108 (1988); Neta et al., Blood, 72:1093 (1988); Trinchieri. "Effects of TNF and Lymphotoxin on the Hematopoietic System." Immunology Series, 56:289-313 (1992); Neta et al., Cancer Cells, 3:391-396 (1991)]. Other references discussing such effects of TNF are described more particularly below.

Neta et al., J. Immunol., 136:2483 (1987) teach that administration of recombinant human TNF protected lethally irradiated normal mice from death.

Slordal et al. teach that single intravenous administration of murine TNF to BALB/c mice 20 hours before sublethal total body irradiation protected against radiation induced neutropenia and accelerated regeneration of hematopoietic precursors and normalization of peripheral blood cell counts. [Slordal et al., J. Immunol., 142:833 (1989); Slordal et al., Eur. J. Haematol., 43:428-434 (1989)].

Urbaschek et al. report that TNF reduced the lethality rate of an LD 75/30 (dose of radiation that kills 75% of mice in 30 days) to an LD 40/30 when injected in C3H/HeJ mice either 24 hours before or after exposure to whole-body radiation. [Urbaschek et al., Lymphokine Res., 6:179 (1987)].

Sersa et al. also report on the effect of administering recombinant human TNF with ionizing radiation. [Sersa et aL, Int. J. Cancer, 42:129.134(1988)]. Specifically, Sersa et al. examined the effects of the TNF on murine tumors in mice when multiple intravenous TNF injections were administered after local tumor irradiation. Sersa et al. report that the TNF and local tumor irradiation caused a delay in tumor growth. [Id.]. Reduced radiation damage to bone marrow progenitor cells was also observed. [Id.].

Neta et al., Fed. Proc., 46:1200 (abstract) (1987) describe the radioprotective effects of IL-1 and TNF. Neta et al. teach that following intraperitoneal administration, 75-fold higher doses of TNF than of IL-1 were required to obtain significant radioprotection, and that even at optimal doses, TNF was less effective than IL-1 in protecting against increasing doses of radiation. [Id.].

Some investigators have reported that under certain conditions, TNF may sensitize particular tumor cells to radiation. Hallahan et al. teach that addition of TNF to SCC-61 tumor cells *in vitro* 4-8 hours prior to irradiation enhanced tumor cell killing. [Hallahan et al., "The Effects of Cytokines on Tumor Cells," Important Advances in Oncology, pp. 75-76 (1993); in Intl. J. Radiation Oncology, Biology, Physics, 27:Suppl. 1, No. 184, Abstract No. 94 (1993), Hallahan et al. also describe administering TNF to patients 4 hours prior to irradiation on consecutive days for at least two weeks]. TNF pretreatment may also render the human leukemic cell lines HL-60, K562, and U937 more sensitive to radiation. [Wong et al., in Eicosanoids and Other Bioactive Lipids in Cancer. Inflammation and Radiation Injury, S. Nigam, ed., (1993) Ch. 70 pp. 353-357]. Other investigators, however, observed no such sensitizing effect by TNF. Sersa et al. report that TNF did not affect tumor cell radiosensitivity. [Sersa et aL, supra].

The effects of TNF in combination with certain chemotherapy drugs have been the subject of further reports. Seibel et al., J. Immunotherapy, 16:125-131 (1994) describe administration of TNF in combination with Actinomycin D to pediatric cancer patients. Some investigators have hypothesized that it may be possible to enhance the therapeutic effects of the chemotherapy agent by simultaneous clinical use of recombinant TNF, and decrease the effective dosage of the chemotherapy agent so that side effects be reduced. [Nakata et al., Jap. J. Cancer Chemo., 13:3186-3193 (1986)].

To date, the use and effects of LT in cancer treatments have not been fully examined. In particular, there is little known about the therapeutic effects of LT and radiation therapy or chemotherapy. Pretreatment of normal C3H/HeJ mice by intraperitoneal injection of LT was reported to increase survival rate following lethal doses of radiation. [Wong et al., Eicosanoids and Other Bioactive Lipids in Cancer, Inflammation and Radiation Injury, supra].

Pluripotent stem cells found primarily in the bone marrow of mammals have the potential to give rise to different types of blood cells which circulate in the peripheral blood. [Dexter et al., Ann. Rev. Cell Biol., 3:423-441 (1987)]. The pluripotent stem cells differentiate into various cell lineages through maturational stages, thereby giving rise to committed blood cell types. One cell lineage differentiated in the bone marrow is the megakaryocytic lineage.

Regulation ofmegakaryocytopoiesis and thrombocyte production has been reviewed by Mazur, Exp. Hemat., 15:248 (1987) and Hoffman, Blood, 74:1196-1212 (1989). At least three classes of megakaryocytic progenitor cells have been identified: (1) burst forming unit megakaryocytes (BFU-MK); (2) colony-forming unit megakaryocytes (CFU-MK); and light density megakaryocyte progenitor cells (LD-CFU-MK). Maturation of megakaryocytes has also been separated into stages based on standard morphologic criteria. The earliest recognizable member of the cells is the megakaryoblast. The intermediate form of the cells is referred to as the promegakaryocyte or basophilic megakaryocyte. The later form of the cells is referred to as the mature (acidophilic, granular or platelet-producing) megakaryocyte. The mature megakaryocyte extends filaments of cytoplasm into sinusoidal spaces where they detach and fragment into individual thrombocytes or "platelets." [Williams et al., Hematology, 1st Ed., McGraw-Hill, Inc., New York. New York (1972)].

Megakaryocytopoiesis is believed to involve several regulatory factors. [Williams et al., Br. J. Haematol., 52:173 (1982); Williams et al., J. Cell Phys., 110:101 (1982)]. The early stage of megakaryocytopoiesis is believed to be mitotic, involving primarily cell proliferation and colony initiation from CFU-MK but not affected by platelet count. [Burstein et al., J. Cell Phys., 109:333 (1981); Kimura et al., Exp. Hematol., 13:1048 (1985)]. The later stage of maturation is primarily non-mitotic, involving nuclear polyploidization and cytoplasmic maturation. [Odell et al., Blood, 48:765 (1976); Ebbe et al., Blood, 32:787 (1968)].

Thrombocytes generally circulate in the blood and play an important role in blood clotting and the body's response to injury. Decreases in the circulating levels of thrombocytes in the blood can result from various pathological conditions and therapies. Thrombocytopenia, for instance, can result from impaired production of thrombocytes by the bone marrow, thrombocyte sequestration in the spleen, and increased destruction of thrombocytes by radiation therapy or chemotherapy. Patients receiving large volumes of rapidly administered blood products can also develop thrombocytopenia due to dilution of the blood. Thrombopoietic disorders are further described in Schafner, "Thrombocytopenia and Disorders of Platelet Function," Internal Medicine, 3rd Ed., Hutton et al., Eds. (1990).

Certain cytokines and growth factors have been identified as inducing thrombocyte production and megakaryocyte growth. Cytokines reported to have megakaryocyte stimulating activity (MK-CSA) include interleukin-3 (IL-3) [Williams et al., Leukemia Res., 9:1487-1491 (1985)], interleukin-6 (IL-6) [Bruno et al., Exp. Hemat., 17:1038-1043 (1989)], granulocyte-macrophage colony stimulating factor (GM-CSF) [Ishibashi et al., Blood, 75:1433-1438 (1990)], interleukin-11 (IL-11) [Xu et al., Blood, 83:2023-2030 (1994)], erythropoietin (EPO) [Sakaguchi et al., Exp. Hemat., 15:1023-1034 (1987)]. and interleukin-12 (IL-12) [Waldburger et al., Exp. Hemat., 22:479a (1994) (suppl.)]. Other cytokines have been reported to modulate platelet development when combined with growth factors possessing established MK-CSA. These cytokines include IL-1α [Gordon et al., Blood, 80:302-307 (1992)] and Leukemia Inhibitory Factor (LIF) [Metcalf et al., "Actions of leukemia inhibitory factor on megakaryocyte and platelet formation." Ciba Foundation Symposium]. The cMpl ligand has also been reported to be a stimulator of megakaryocytopoiesis and thrombopoiesis. [de Sauvage et al., Nature, 369:533-538 (1994); Lok et al., Nature, 369:565-568 (1994); Kaushansky et al., Nature. 369:568-571 (1994)]. Further, a synthetic protein reported to stimulate thrombopoiesis is PIXY 321. [van de Ven et al., Exp. Hematol., 20:743-751 (1992); Williams et aL, Blood, 82:366a (1993) (suppl.); Collins et al., Blood, 82:366a (1993) (suppl.)}. PIXY 321 is a fusion protein composed of GM-CSF and IL-3 linked by a synthetic peptide chain [Williams, et al., Cancer, 67:2705-2707 (1991)].

IL-3 is believed to principally affect the differentiation (earliest) phase of the thrombocytopoiesis process. [Moore et al., Blood, 78:1 (1991); Sonoda et al., Proc. Natl. Acad. Sci. USA, 85:4360 (1988)]. IL-6 has been shown to induce megakaryocyte progenitor cell proliferation as well as maturation in murine and primate models, although some investigators have observed IL-6 effects only on cell maturation. The interaction between IL-3 and IL-6 is currently unclear, with some reports indicating that the MK-CSA of IL-3 is mediated by IL-6 since antibodies against murine IL-6 were found to abrogate the MK-CSA of murine IL-3. [Lotem et al., Blood, 74:1545-1551 (1989)]. Other investigators have reported that neutralizing antibodies to IL-6 alone do not diminish the MK-CSA of serum prepared from aplastic patients. and hypothesize a less crucial role for IL-6 in megakaryocytopoiesis. Nevertheless, a number of studies have demonstrated a stimulatory effect of IL-6 when administered to lethally irradiated animals. [Burstein et al., Blood, 80:420-428 (1992); Herodin et al., Blood, 80:68-74 (1992)].

In patients receiving high dose chemotherapy for metastatic sarcoma and lung cancer, IL-6 has reduced the decline in platelet count and hastened the recover to baseline levels. [Demetri et al., Blood, 82:367a (1993) (suppl.); Crawford et al., Blood, 82:367a (1993) (suppl.)]. Autologous bone marrow transplant recipients treated with IL-6 have been reported to experience a relatively rapid return of circulating platelets and a shorter period of platelet transfusion dependence than matched controls. [Fay et al., Bload, 82:431 a (1993) (suppl.)].

### Summary of the Invention

The object of the invention is defined in the claims.

Applicant has surprisingly found that administration of LT and thrombopoietin provides enhanced maturation of megakaryocytes and production of thrombocytes. Reduced levels of thrombocytes in the blood can jeopardize the health of individuals. As discussed in the Background ofthe Invention, dangerously low levels of thrombocytes in the blood can be the result of a variety of pathological conditions, as well as chemotherapy and radiation therapy. Accordingly, the administration of LT and thrombopoietin can be useful in assisting to re-establish a damaged or diminished hematopoietic system.

### Brief Description of the Drawings

Figure 1 is a graph showing the effect of LT and Doxorubicin on survival of normal rats.

Figures 2A-2B are photographs comparing LT and Doxorubicin treated rat bone marrow (Figure 2B) to control rat bone marrow (treated only with Doxorubicin) (Figure 2A).

Figure 3 is a bar diagram comparing the effects of Doxorubicin. LT. TNF. LT and Doxorubicin, and TNF and Doxorubicin on viability of U-937 human histiocystic lymphoma cells *in vitro*.

Figure 4 is a photograph of crystal violet-stained ME 180 cervical carcinoma cells cultured *in vitro* with Doxorubicin, LT, medium only (control) or LT and Doxorubicin.

Figure 5 is a graph showing the effect of LT (10 µg/0.5 ml administered on -2 Days and-1 Day prior to radiation on Day 0) on survival of radiated (750 cGy) normal BALB/c mice.

Figure 6 is a graph showing the effect of LT on survival of normal BALB/c mice radiated with varying doses of ¹³⁷Cs gamma-ray whole body radiation.

Figure 7 is a graph showing the effects of LT and TNF on survival of radiated (750 cGy) tumor bearing BALB/c mice.

Figure 8 is a graph showing the effect of LT on survival of tumor bearing BALB/c mice radiated with varying doses of ¹³⁷Cs gamma-ray whole body radiation.

Figure 9 is a bar diagram showing the effect of LT treatment (prior to radiation) in reducing tumor size in radiated (750 cGy) tumor bearing BALB/c mice.

Figure 10 is a bar diagram showing the effect of LT on tumor cell viability in radiated (1500 cGy) tumor bearing nude mice.

Figure 11 shows photographs of clonogenic assay culture plates containing either control or LT treated MDA 231 breast adenocarcinoma tumor cells stained with crystal violet

Figure 12A is a bar diagram showing the effect of LT, radiation, and LT and radiation on tumor weight (grams) of primary human colon tumors in radiated (1500 cGy) tumor bearing nude mice; Figure 12B is a bar diagram showing the effects on total cell yield; Figure 12C is a bar diagram showing the effects on cell viability; Figure 12D is a bar diagram showing the effects on numbers of colony forming cells.

Figure 13 is a graph showing the effect of LT on numbers of colony forming cells in bone marrow of tumor bearing nude mice radiated with varying doses of ¹³⁷Cs gamma-ray whole body radiation.

Figure 14 is a graph showing the effect of LT on circulating platelet levels in radiated (750 cGy) normal C57BL/6 mice.

Figure 15 is a graph showing the effect of LT and TPO332 on recovery of circulating platelet levels in radiated (750 cGy) normal C57BL/6 mice through Day 17 (post radiation treatment).

Figure 16 is a graph showing the effect of LT and TP0332 on recovery of circulating platelet levels in radiated (750 cGy) normal C57BL/6 mice through Day 28 (post radiation treatment).

Figure 17 is a graph showing the effect of LT on recovery of circulating RBC levels in radiated (750 cGy) normal C57BL/6 mice through Day 28 (post radiation treatment).

Figure 18 is a graph comparing the effects of varying doses of LT and TPO332 on recovery of circulating platelet levels in radiated (750 cGy) normal C57BL/6 mice through Day 28 (post radiation treatment).

Figure 19 is a graph showing a dose response curve for LT administered in combination with 0.3 µg TPO332 to radiated (750 cGy) normal C57BL/6 mice.

### Detailed Description of the Preferred Embodiments

### 1. Definitions

As used herein, the term "lymphotoxin" ("LT") refers to a polypeptide having a region demonstrating substantial structural amino acid homology with at least a portion of the sequence comprising amino acids 1-171 of human lymphotoxin. The DNA sequence encoding human lymphotoxin and its corresponding amino acid sequence are published in EP 164,965 A2 and Gray et al., Nature, 312:721-724 (1984). Substantial structural amino acid homology generally means that greater than about 60 percent, and usually greater than about 70 percent of the amino acid residues in the polypeptide are the same or conservative substitutions for the corresponding residues in the sequence for human lymphotoxin. The term lymphotoxin includes the mature, pre, pre-pro, and pro forms of the molecule, either purified from a natural source, chemically synthesized or recombinantly produced. The lymphotoxin of the invention includes leucyl amino-terminal lymphotoxin and histidyl amino-terminal lymphotoxin (such as described in EP 164,965 A2), lymphotoxin aggregates (such as trimers), and lymphotoxin variants including (a) fusion proteins wherein a heterologous polypeptide or protein is linked to the amino and/or carboxyl-terminal amino acids of lymphotoxin, (b) lymphotoxin fragments, especially fragments of pre lymphotoxin in which any amino acid between -34 and +23 is the amino-terminal amino acid of the fragment, (c) lymphotoxin mutants wherein one or more amino acid residues are substituted, inserted or deleted, and (d) methionyl or modified methionyl (such as formyl methionyl or other blocked methionyl species) amino-terminal derivatives. The term lymphotoxin specifically includes the lymphotoxin species disclosed in EP 164,965 A2, published December 18, 1985.

Lymphotoxin as defined herein specifically excludes human tumor necrosis factor (TNF-α) or its animal analogues [such as described by Pennica et al., Nature, 312:724-729 (1984) and Aggarwal et al., J. Biol. Chem., 260:2345-2354 (1985)].

The term "thrombopoietin" refers generally to a polypeptide or protein which is capable of, directly or indirectly, stimulating proliferation, differentiation, or maturation of megakaryocytes or progenitor cells thereof. The term thrombopoietin specifically includes c-Mpl ligand [see, e.g., de Sauvage et al., supra; Lok et al., supra; Kaushansky et al., supra]. The term thrombopoietin includes the mature, pre-pro, and pro forms of the polypeptide or protein, either isolated or purified from a natural source, chemically synthesized, or recombinantly produced. The term includes native full-length mature thrombopoietin, as well as derivatives, fragments, alleles, amino acid variants, glycosylation variants, isoforms, and analogues thereof, including those described in co-owned and co-pending application Pub. No. WO95/18858, published 13 July 1995, capable of, directly or indirectly, stimulating proliferation, differentiation, or maturation of megakaryocytes or progenitor cells thereof.

The terms "amino acid" and "amino acids" refer to all naturally occurring L-α-amino acids. This definition is meant to include norleucine, omithine, and homocysteine. The amino acids are identified by either the single-letter or three-letter designations:

| | |
|---|---|
| Asp D aspartic acid | Ile I isoleucine |
| Thr T threonine | Leu L leucine |
| Ser S serine | Tyr Y tyrosine |
| Glu E glutamic acid | Phe F phenylalanine |
| Pro P proline | His H histidine |
| Gly G glycine | Lys K lysine |
| Ala A alanine | Arg R arginine |
| Cys C cysteine | Trp W tryptophan |
| Val V valine | Gln Q glutamine |
| Met M methionine | Asn N asparagine |

Substitution of an amino acid with an amino acid(s) having a side chain that is similar in charge and/or structure to that of the native molecule is referred to as a conservative substitution, and would not be expected to substantially alter either the structure of the backbone of the molecule or the charge or hydrophobicity of the molecule in the area of the substitution.

The terms "treating," "treatment," and "therapy" refer to curative therapy, prophylactic therapy, and preventative therapy.

The terms "cancer" and "cancerous" refer to or describe the physiological condition in mammals that is typically characterized by unregulated cell growth. Examples of cancer include but are not limited to, carcinoma, lymphoma, blastoma. sarcoma, and leukemia. More particular examples of such cancers include squamous cell carcinoma, small-cell lung cancer, non-small cell lung cancer, pancreatic cancer, glioblastoma multiforme. cervical cancer, bladder cancer, hepatoma, breast cancer, colon carcinoma, and head and neck cancer.

The term "megakaryocyte maturation" refers to a process involving differentiation of megakaryoblasts and promegakaryocytes or basophilic megakaryocytes into mature, platelet-producing cells. Megakaryocyte maturation is typically accompanied by cellular changes. such as increased ploidy and demarcation of membranes, and can be observed and quantitated, for instance, by ploidy analysis and microscopic analysis.

The term "thrombocytopenia" refers to a physiological condition typically characterized by a thrombocyte level below about 150 x 10⁹/iter blood.

The term "mammal" refers to any mammal classified as a mammal. including humans, cows, horses, dogs, cats, rats and mice. In a preferred embodiment of the invention, the mammal is a human.

### 2. Methods and Compositions of the Invention

The present invention provides methods and compositions for treating cancer which include lymphotoxin, referred to hereinafter as "LT". The LT useful in the practice of the present invention can be prepared in a number of ways. For instance, the LT can be prepared using an isolated or purified form of LT from natural sources or from cell lines. Methods of isolating or purifying LT are known in the art and are described, for example, in U.S. Patent No. 4,920,196. Using the methods described in U.S. Patent No. 4,920,196, human LT can be purified to a specific activity of at least about 10⁶ units/mg protein. Alternatively, LT can be chemically synthesized or produced using recombinant techniques known in the art and described further in U.S. Patent No.4.959,457 and EP 164,965 A2. The present invention is not limited to use of human LT. Other mammalian species of LT, such as murine or rabbit, may be employed. In a preferred embodiment, human LT is employed for treating humans having cancer.

The LT is preferably administered to the mammal in a pharmaceutically-acceptable carrier. Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the formulation to render the formulation isotonic. Examples of the pharmaceutically-acceptable carrier include saline, Ringer's solution and dextrose solution. The pH of the solution is preferably from about 5 to about 8, and more preferably from about 7.4 to about 7.8. It will be apparent to those persons skilled in the art that certain carriers may be more preferable depending upon, for instance, the route of administration and concentration of LT being administered.

The LT can be administered to the mammal by injection (e.g., intravenous. intraperitoneal, subcutaneous. intramuscular), or by other methods such as infusion that ensure its delivery to the bloodstream in an effective form. Local or intravenous injection is preferred.

Effective dosages and schedules for administering LT may be determined empirically, and making such determinations is within the skill in the art. It is presently believed that an effective dosage or amount of human LT in mice and rats is in the range of about 50 µg/kg/day to about 500 µg/kg/day. Interspecies scaling of dosages can be performed in a manner known in the art, e.g., as disclosed in Mordenti et al., Pharmaceut, Res., 8:1351 (1991). Those skilled in the art will understand that the dosage of LT that must be administered will vary depending on, for example, the mammal which will receive the LT, the route of administration, and other drugs being administered to the mammal. The skilled clinician will understand, for instance, that lower dosages of human LT will likely be effective in humans as compared to human LT in mice and rats. Further, the skilled clinician will understand that intravenous administration of LT will likely require lower dosages as compared to intraperitoneal administration.

The one or more other anti-cancer therapies administered to the mammal include but are not limited to, chemotherapy and radiation therapy. Anti-cancer therapies such as immunoadjuvants and cytokines may also be employed, including interleukins (e.g., IL-1, IL-2, IL-3, IL-6), leukemia inhibitory factor, interferons, TGF-beta, erythropoietin and thrombopoietin. As used in the present application, the term "one or more other anti-cancer therapies" specifically excludes LT and TNF.

Chemotherapy contemplated by the invention includes chemical substances or drugs which are known in the art and are commercially available, such as Doxorubicin, 5-Fluorouracil, Cytosine arabinoside ("Ara-C"), Cyclophosphamide, Thiotepa, Busulfan, Cytoxin, Taxol, Methotrexate. Cisplatin, Melphalan, Vinblastine and Carboplatin. Preparation and dosing schedules for such chemotherapy may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore. MD (1992).

The chemotherapy is preferably administered in a pharmaceutically-acceptable carrier, such as those described above for LT. The mode of administration of the chemotherapy may be the same as employed for the LT or it may be administered to the mammal via a different mode. For example, the LT may be injected while the chemotherapy is administered orally to the mammal. Administration of LT and chemotherapy to a mammal is described in further detail in Example 1 below.

Radiation therapy can be administered to the mammal according to protocols commonly employed in the art and known to the skilled artisan. Such therapy may include cesium, iridium, iodine, or cobalt radiation. The radiation therapy may be whole body irradiation, or may be directed locally to a specific site or tissue in or on the body, such as the lung, bladder, or prostate. Typically, radiation therapy is administered in pulses over a period of time from about 1 to about 2 weeks. The radiation therapy may, however. be administered over longer periods of time. For instance, radiation therapy may be administered to mammals having head and neck cancer for about 6 to about 7 weeks. Optionally, the radiation therapy may be administered as a single dose or as multiple, sequential doses.

The present invention also provides the use of lymphotoxin and thrombopoeitin in the preparation of a medicament for enhancing stimulation of megakaryocyte maturation or thrombocyte production in a mammal. LT employed in the methods is described above and in further detail in the Examples below. The thrombopoietin useful in the practice of the present invention can be prepared in a number of ways. For instance, the thrombopoietin can be isolated or purified from natural sources [see, e.g., de Sauvage et al., supra]. Alternatively, the thrombopoietin can be chemically synthesized and prepared using recombinant DNA techniques known in the art and described in further detail in de Sauvage et al., supra; Lok et al., supra. The thrombopoietin may be from human or any non-human species. For instance, a mammal may have administered thrombopoietin from a different mammalian species (e.g., mice can be treated with human thrombopoietin). Preferably, however, the mammal is treated with homologous thrombopoietin (e.g., humans are treated with human thrombopoietin) to avoid potential immune reactions to the thrombopoietin.

The present invention includes methods for enhancing stimulation of megakaryocyte maturation or thrombocyte production *in vitro*. In accordance with the method of the invention for stimulating megakaryocyte maturation *in vitro,* bone marrow cells or cell samples suspected of containing megakaryoblasts, promegakaryocytes and/or basophilic megakaryocytes are provided and placed in a cell culture medium. The cells are then cultured in the presence of LT and thrombopoietin.

Suitable tissue culture media are well known to persons skilled in the art and include, but are not limited to, Minimal Essential Medium ("MEM"), RPMI-1640, and Dulbecco's Modified Eagle's Medium ("DMEM"), These tissue culture medias are commercially available from Sigma Chemical Company (St. Louis, MO) and GIBCO (Grand Island. NY). The cells are then cultured in the cell culture medium under conditions sufficient for the cells to remain viable and grow. The cells can be cultured in a variety of ways, including culturing in a clot, agar, or liquid culture.

The cells are cultured in the presence of effective amounts of LT and thrombopoietin. The LT and thrombopoietin may be added to the culture at a dose determined empirically by those in the art without undue experimentation. The concentration of LT and thrombopoietin in the culture will depend on various factors, such as the conditions under which the cells and LT and thrombopoietin are cultured. The specific temperature and duration of incubation, as well as other culture conditions, can be varied depending on such factors as, e.g., the concentration of the LT and thrombopoietin, and the type of cells and medium. Those skilled in the art will be able to determine operative and optimal culture conditions without undue experimentation. Maturation of megakaryocytes, and thrombocyte production, in the cultures can be determined by various assays known in the art, such as those described in Williams et al., Leukemia Res., 9:1487-1491 (1985): Bruno et al., Exp. Hematol., 17:1038-1043 (1989); Ishibashi et al., Blood, 75:1433-1438 (1990): Xu et al., Blood, 83:2023-2030 (1994); Sakaguchi et al., Exp. Hematol., 15:1023-1034 (1987)].

It is contemplated that using LT and thrombopoietin to enhance stimulation of megakaryocyte maturation or thrombocyte production *in vitro* will be useful in a variety of ways. For instance, megakaryocytes cultured *in vitro* in the presence of LT and thrombopoietin can be infused into a mammal suffering from reduced levels of platelet-forming cells.

For enhancing stimulation of megakaryocyte maturation or thrombocyte production in a mammal, effective amounts of LT and thrombopoietin are to be administered to the mammal. It is contemplated that the LT-thrombopoietin treatment may be administered at the time of, or after, administering to the mammal therapy, such as radiation therapy or chemotherapy, which can adversely affect circulating blood thrombocyte levels or bone marrow containing megakaryocyte progenitor cells. The LT-thrombopoietin treatment may also be administered prophylactically so as to avoid a decrease in circulating blood thrombocyte levels or reduce or prevent bone marrow damage.

Modes and means of administering LT are described above. The thrombopoietin may be formulated in pharmaceutically-acceptable carriers and administered by means such as described above for LT. It will be apparent to those persons skilled in the art that certain carriers may be more preferable depending upon, for instance, the route of administration and concentration of LT and thrombopoietin being administered. Further, it is contemplated that the LT and thrombopoietin may be included in the same carrier, or alternatively, may be formulated in a different or separate carrier. The LT and thrombopoietin are preferably both administered to the mammal by injection (e.g., intravenous, intraperitoneal, subcutaneous, intramuscular) but may be administered by different means, i.e., the LT may be injected while the thrombopoietin is administered orally or intranasally.

Effective dosages and schedules for administering LT and thrombopoietin may be determined empirically, and making such determinations is within the skill of the art. As described above, an effective amount of human LT in mice and rats is in the range of about 50 µg/kg body weight/day to about 500 µg/kg body weight/day, and interspecies scaling of dosages can be performed in a manner known in the art, e.g., as disclosed in Mordenti et al., Pharmaceut. Res. 8:1351 (1991). An effective daily regimen of thrombopoietin is believed to be about 0.1 mg/kg body weight to about 100 mg/kg body weight, preferably about 0.1 mg/kg body weight to about 50 mg/kg body weight, and more preferably, about 1 mg/kg body weight to about 5 mg/kg body weight. It is understood by those skilled in the art that the dose of LT and thrombopoietin that must be administered will vary depending on. for example, the mammal which will receive the LT and thrombopoietin, the nature of the medical condition or therapy believed to be responsible for decreased thrombocyte levels, the extent of damage to the blood cell producing tissues, the route of administration, and the identity of any other drugs being administered to the mammal. The effective amounts of each for purposes herein are thus determined by such considerations and must be amounts that enhance stimulation of megakaryocyte maturation or thrombocyte production over the stimulation that is obtained using the same amount of LT or thrombopoietin individually. It will also be understood by the skilled clinician that it may be necessary to give more than one dose of LT and thrombopoietin. Generally, multiple doses of both LT and thrombopoietin will be required for administration.

The LT and thrombopoietin may be administered to the mammal concurrently or sequentially. Preferably, administration is sequential, and more preferably, LT is administered to the mammal prior to the administration of the thrombopoietin. In an even more preferred embodiment. at least 2 doses of LT are administered to the mammal prior to the administration of thrombopoietin. For a mammal receiving chemotherapy or radiation therapy, it is preferred that the LT is administered prior to administration of the chemotherapy or radiation therapy, and that the thrombopoietin is administered after the administration of chemotherapy or radiation therapy. Such LT pretreatment is described in further detail in Example 5.

The invention also provides the use of lymphotoxin and thrombopoeitin in the preparation of a medicament for treating thrombocytopenia in a mammal. In the method, the mammal is first diagnosed as suffering from thrombocytopenia. Making the diagnosis is within the skill in the art. Those skilled in the art will also appreciate that different thrombocyte levels may warrant a thrombocytopenia diagnosis for different mammalian species. The diagnosis is usually made in humans when thrombocyte levels fall below about 150 x 10⁹ thrombocytes/liter of blood. Thrombocytopenia can be the result of a disorder of production, distribution or destruction of thrombocytes or thrombocyte-producing cells. To treat the thrombocytopenia, the LT and thrombopoietin are administered to the mammal according to the modes and schedules of administration described above.

In the aforementioned methods, it is of course contemplated that the LT and thrombopoietin can be administered to a mammal in combination with one or more other biologically or chemically active agents. For instance, the LT and thrombopoietin may be administered in combination with a cytokine, particularly a colony stimulating factor or interleukin. Such colony stimulating factors or interleukins include LIF. G-CSF, GM-CSF. M-CSF, stem cell factors, EPO, IL-1, IL-2, IL-3, IL-5, IL-6, IL-7, IL-8, IL-9, and IL-11.

The megakaryocyte maturation and thrombocyte production can be measured or monitored in various ways. For instance, the maturation and thrombocyte production can be measured using *in vitro* assays. Megakaryocyte progenitor assays are known in the art and can be performed, for example, by culturing the cells in methylcellulose as described by Tanaka et al., Br, J. Haematol. 73:18 (1989). Single cell growth assays can also be performed as described by Williams et al., Cell Tissue Kinetics, 15:483 (1982); Banu et al., Br. J. Haematol., 75:313 (1990); Oon et al., Leukemia Res., 10:403 (1986); Sparrow et al., Leukemia Res., 11:31 (1987).

The maturation and thrombocyte production can also be monitored by peripheral blood or bone marrow analysis. Thrombocyte levels in the circulating blood can be determined by cell count analysis. The cell count analysis may be performed by counting viable cells by trypan blue exclusion. The cells can also be examined morphologically. For example, bone marrow samples can be obtained from the mammal and prepared for microscopy using standard histological techniques known in the art. By staining the cells, one can observe the size, cellular characteristics, and number of megakaryocytes in the marrow sample.

The invention will be more fully understood by reference to the following examples. They should not, however. be construed as limiting the scope of the invention. All reference citations herein are incorporated by reference.

### Examples

Examples 1-4 refer to technical background.

The following Examples describe various *in vitro* and *in vivo* studies using LT. The LT used in these Examples was human histidyl amino-terminal lymphotoxin (approximately 16 kDa and having amino acids 24-171 of human lymphotoxin) produced in accordance with the recombinant methods described in EP 164.965 A2. The expressed LT was precipitated using polyetheneimine (PEI) precipitation and ammonium sulfate precipitation and then purified by column chromatography. The LT was chromatographed sequentially on silica gel, hydroxylapatite, and Sephacryl S-200. The LT was then sterile filtered (using a 22 µm filter), diluted in 0.1 M Tris-HCl buffer (pH 7.8) at a concentration of 0.6 mg/ml, and stored at 4°C.

The TNF used in the Examples was human TNF produced in accordance with the recombinant methods described in EP 168,214 published January 1, 1986 and Pennica et al., Nature, 312:724-729 (1984). The expressed TNF was precipitated using PEI precipitation and purified by column chromatography. The TNF was chromatographed sequentially on silica gel, DEAE fast flow sepharose, carboxymethyl cellulose (CM-52). Mono S cation exchange resin, and Sephacryl S-200. The TNF was then sterile filtered (using a 22 µ*m* filter), diluted in phosphate buffered saline (pH 7.0) at a concentration of 0.5 mg/ml, and stored at 4°C.

The thrombopoietin used in the Examples was recombinant human full length c-Mpl ligand [de Sauvage et al., supra] (referred to herein as "TPO332") described in co-owned and co-pending application Pub. No. WO95/18858. published 13 July 1995. Briefly, the TP0332 was expressed and purified from Chinese hamster ovary (CHO) cells as follows. cDNA corresponding to the entire open reading frame (de Sauvage et al., supra) was obtained by PCR. The PCR product was purified and cloned between two restriction sites (Clal and Sail) of the plasmid pSV15.ID.LL (derived from pSVI5 described in U.S. Patent No. 5,087,572) to obtain the vector pSVI5.ID.LL.MLORF. The vector construct was linearized with NotI and transfected into CHO-DP12 cells (EP 307,247 published March 15, 1989) by electroporation. 10⁷ cells were electroporated in a BRL electroporation apparatus (350 Volts, 330 mF, low capacitance) in the presence of 10, 25 or 50 mg of DNA as described in Andreason, J. Tissue Cult. Meth., 15:56 (1993). The day following transfection, cells were split in DHFR selective media (high glucose DMEM-F12 50:50 without glycine, 2mM glutamine, 2-5% dialyzed fetal calfserum). Ten to fifteen days later, individual colonies were transferred to 96 well plates and allowed to grow to confluency.

To purify and isolate the TPO332, the harvested culture fluid from the cells was applied to a Blue Sepharose 6 Fast Flow column (Pharmacia) at a ratio of approximately 100 L of culture fluid per liter of resin. The column was washed with 3-5 column volumes of sodium phosphate buffer, pH 7.4, followed by 3-5 column volumes of buffer containing 2 M urea. The TP0332 was then eluted with 3-5 column volumes of buffer containing 2 M urea, 1 M NaCl. The eluate was then applied to a Wheat Germ Lectin Sepharose 6MB column (Pharmacia) equilibrated in eluting buffer (0.01M sodium phosphate, pH 7.4, 2M urea, 1M NaCl) at a ratio of from 8-16 ml of eluate per ml of resin, followed by washing with 2-3 column volumes of equilibration buffer. The TPO was eluted with 2-5 column volumes of buffer containing 2M urea, 0.5M.N-acetyl-D-glucosamine. The eluate was then acidified and C₁₂E₈ was added to a final concentration of 0.04%. The resulting pool was applied to a C4 reversed phase column equilibrated in 0.1% trifluroacetic acid (TFA), 0.04% C₁₂E₈ at a load of approximately 0.2 to 0.5 mg protein per ml of resin. The protein was then eluted in a two phase linear gradient of acetonitrile containing 0.1% TFA and 0.04% C₁₂E₈. The C4 pool was then diluted with 2 volumes of 0.01 M sodium phosphate, pH 7.4, 0.15M NaCl, and diafiltered versus approximately 6 volumes of buffer on an Amicon YM or like ultrafiltration membrane having a 10.000 to 30,000 dalton molecular weight cut-off. The diafiltrate was then applied to a Sephacryl S-300 HR column (Pharmacia) equilibrated in buffer (0.01M sodium phosphate, pH 7.4,0.15M NaCI) containing 0.01% Tween-80 and chromatographed. The resulting pool was filtered using a 22 *µm* filter and stored at 2-8°C.

The cell lines referred to in the Examples by "ATCC" numbers were obtained from the American Type Culture Collection, Rockville, MD.

### Example 1: Effects of LT and Chemotherapy in Vivo

### A. Effect on Survival

Seven day old rats (obtained from Harlan Sprague Dawley) were treated with LT and Doxorubicin. Doxorubicin (also referred to as Adriamycin®) (purchased from Adria Pharmaceuticals) was injected intraperitoneally (0.1 ml/rat) for seven consecutive days (Day 0 to Day 6) at a dose of 2 mg/kg/day. Rats were injected intraperitoneally with LT at a dose of 1 µg/0.1 ml/rat/day. The LT was administered at -2 Days and -1 Day prior to commencing Doxorubicin treatment on Day 0. Administration of LT continued from Day 0 through Day 6. Control animals were treated similarly except that 0.1ml sterile phosphate buffered saline ("PBS") was injected intraperitoneally instead of LT. Survival was monitored daily until Day 15.

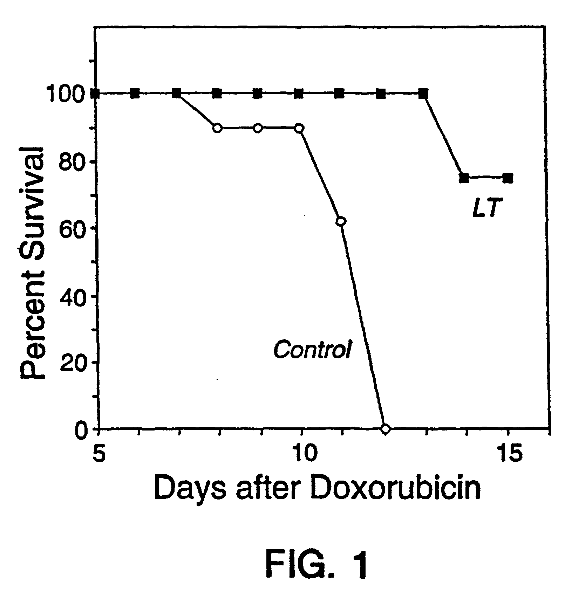

The results are shown in Figure 1. The LT-Doxorubicin treated group showed 100% survival at Day 12 and 80% survival on Day 15. The control group had 0% survival at Day 12.

### B. Effect on Bone Marrow

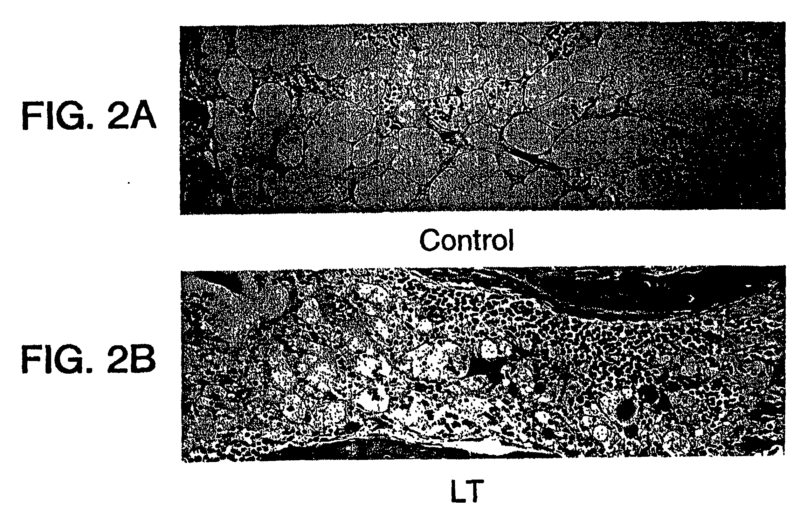

One of the LT-Doxorubicin treated rats and one of the control rats described in Example 1, Section A above were sacrificed on Day 9. Bone stemums from the rats were dissected, and marrow cells from the stemums were fixed with 10% neutral-buffered Formalin and embedded in paraffin wax. Approximately 4 µm sections of the embedded cells were stained with hematoxylin and eosin on microscope slides. Histological analysis showed that the rat receiving LT and Doxorubicin had granulocyte progenitor cells and megakaryocytes that were protected from the chemotherapy induced toxicity whereas very few cells were found in sternum marrow obtained from the control rat. (See Figure 2).

### C. Effect on Alopecia

Eight day old rats (obtained from Harlan Sprague Dawley) were treated with LT and cytosine-arabinoside ("Ara-C") in accordance with the protocol outlined in Example 1, Section A above except that Ara-C (purchased from Upjohn) was injected intraperitoneally (0. 1ml/rat) at a dose of 20 mg/kg/day from Day 0 to Day 6. Control animals were similarly treated except that 0.1ml sterile PBS was injected instead of LT.

On Day 15, the degree of alopecia observed for the control and LT-Ara-C treated animals was rated on a scale of 0 to 4+. with 0 representing no observable hair loss and 4+ representing substantial balding of the animal. The results are shown in Table 1 below. Enhanced protection from alopecia occurred in the LT-Ara-C treated group with 9/10 animals demonstrating no observable hair loss.

**Table 1**

| | **DEGREE OF ALOPECIA** | | | | |
|---|---|---|---|---|---|
| **TREATMENT** | **0** | **1+** | **2+** | **3+** | **4+** |
| Contol(Ara-C only) | | 1 | 1 | 5 | 3 |
| LT and Ara-C | 9 | 1 | | | |

### Example 2: Effects of LT and Chemotherapy in Vitro

### A. Effect on U-937 Tumor Cells

U-937 cells (human histiocystic lymphoma cell line. ATCC No. CRL 1593) were cultured in RPMI-1640 medium in 96 well tissue culture plates at a concentration of 10⁵ cells/well. The cell cultures were treated with (1) Doxorubicin (50 *µg*/ml); (2) LT (0.1 µg/ml); (3) TNF (0.1 µg/ml); (4) LT and Doxorubicin; or (5) TNF and Doxorubicin. Control cultures contained RPMI-1640 medium.

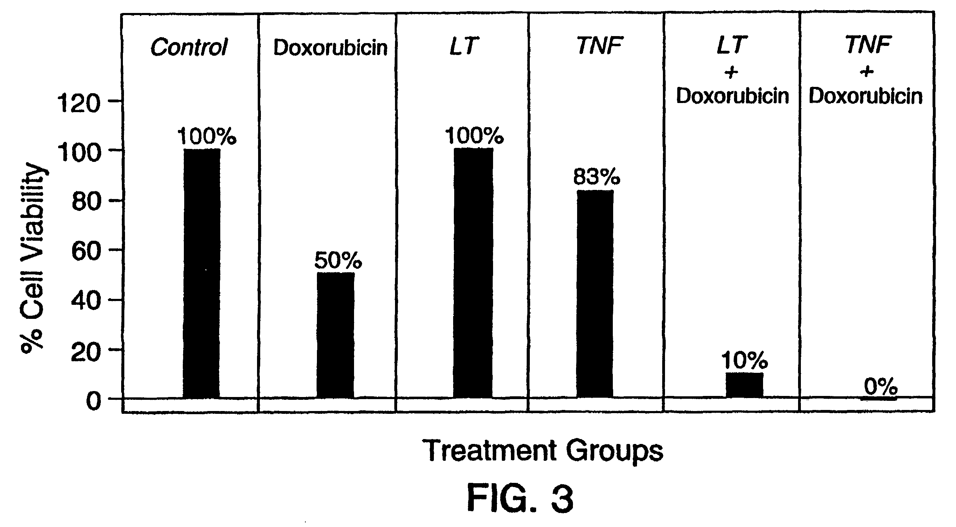

The tissue culture plates containing the test cultures and control cultures were then incubated at 37° C for 24 hours. After the incubation, the cells were tested for viability using trypan blue dye (diluted to 0.4% in PBS) exclusion. The results are shown in Figure 3. Both LT and TNF enhanced sensitivity ofthe U-937 cells to the Doxorubicin.

### B. Effect on ME-180 Tumor Cells

ME 180 cells (human epidermoid carcinoma cell line, ATCC No. HTB 33) were cultured in RPMI-1640 medium in 96 well tissue culture plates at a concentration of 10⁵ cells/ml. The cell cultures were treated with either 0.1 µg/ml LT and varying dilutions of Doxorubicin (200 µg/ml, 100 µg/ml, 50 µg/ml, and 25 µg/ml and 2-fold dilutions thereof) or the varying dilutions of Doxorubicin alone. RPMI-1640 medium or LT (0.1 µg/ml) was added to the control cultures. The tissue culture plates containing the test cultures and control cultures were then incubated at 37°C for 30 hours. Following incubation, the cells were stained with crystal violet (0.5% crystal violet in 20% methanol).

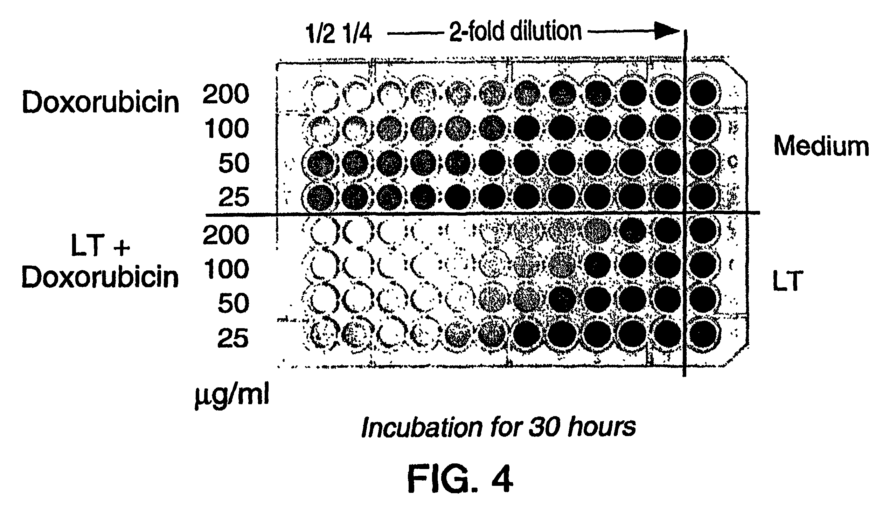

The stained cultures showed that co-cultivation of the ME 180 cells with LT and Doxorubicin enhanced cell killing significantly. (Figure 4). Viable cells were detected in the cell cultures treated with Doxorubicin alone at concentrations of Doxorubicin as high as 50-100 µg/ml. In contrast, no viable cells were identified in the cultures treated with LT and Doxorubicin at concentrations of Doxorubicin as low as 3-6 µg/ml. (Figure 4).

Similarly conducted experiments using BxPC-3 cells (human pancreatic adenocarcinoma cell line, ATCC No. CRL 1687), Su.86.86 cells (human pancreatic carcinoma cell line, ATCC No. CRL 1837), A549 cells (human lung carcinoma cell line, ATCC No. CCL 185), and L929 cells (murine lung fibroblast cell line. ATCC No. CCL 1) also showed that LT and Doxorubicin treatment resulted in enhanced killing of the tumor cells and that Doxorubicin was effective at lower concentrations when LT was added to the cell cultures. LT (0.1 µg/ml) alone was ineffective for killing the tumor cells. Further, Doxorubicin (below 50 µg/ml) alone was ineffective for killing the tumor cells. When such concentrations of LT and Doxorubicin were combined, however, no viable cells could be detected in the cultures after incubation.

### Example 3: Effects of LT and Radiation

### Therapy in Vivo

### A. Radioprotection in Normal Mice

Normal 8 week old BALB/c mice (purchased from Harlan Sprague Dawley) were treated with intraperitoneal injections of LT (10 µg/0.5ml/mouse) at -2 Days and -1 Day prior to radiation on Day 0. Radiation was conducted using ¹³⁷Cs gamma-ray whole body radiation. Groups of mice were radiated with varying doses of radiation ranging from 500 cGy to 800 cGy. Control mice were similarly treated except that 0.5ml sterile PBS was injected instead of LT.

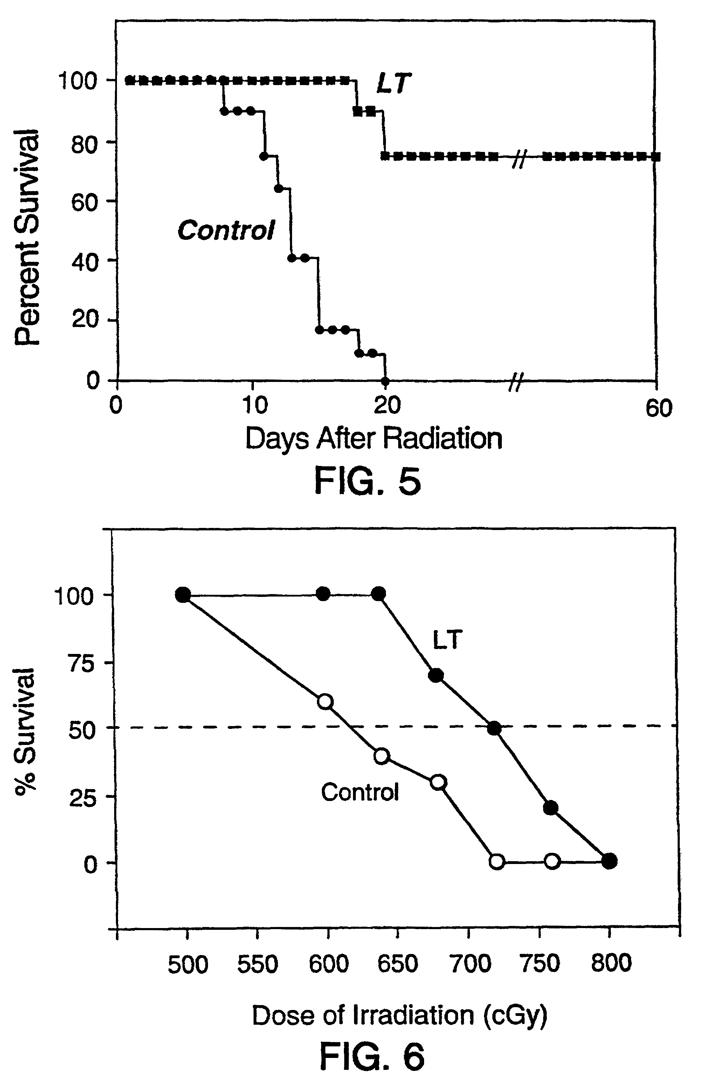

Figure 5 shows that at 750 cGy, 80% of the mice receiving LT treatment survived up to 60 days post radiation. 100% of the control animals were dead on Day 20. (Figure 5). Similar results were observed for nude mice, C57BL/6, and C3H/HeJ mice (radiated with lethal doses of radiation). thus indicating that the radioprotective effect of LT was not strain specific, (data not shown).

Figure 6 is a graph showing the effects of the LT and varying doses of radiation at Day 30 post radiation. The LT treated animals tolerated an approximately 20% increase in radiation dose at the LD50 level. (Figure 6).

### B. Radioprotection in Tumor Bearing Mice

The radioprotective effects of LT were also examined in tumor bearing BALB/c mice. BALB/c mice (purchased from Harlan Sprague Dawley) were injected subcutaneously with murine Meth-A fibrosarcoma cells (2 x 10⁵ cells/mouse) [obtained from Lloyd Old, Memorial Sloan-Kettering Cancer Center. New York. NY; see also, Carswell et al., Proc. Natl. Acad. Sci., 72:3666-3670 (1970)]. The tumors were then allowed to grow for 10 days. Either LT (10 µg/0.5ml/mouse) or TNF (10 µg/0.5ml/mouse) was then injected intraperitoneally in the tumor bearing mice at -2 Days and -1 Day prior to radiation on Day 0. The mice were radiated on Day 0 using 750 cGy ¹³⁷Cs gamma-ray whole body radiation. Survival was then observed up to 40 days post radiation.

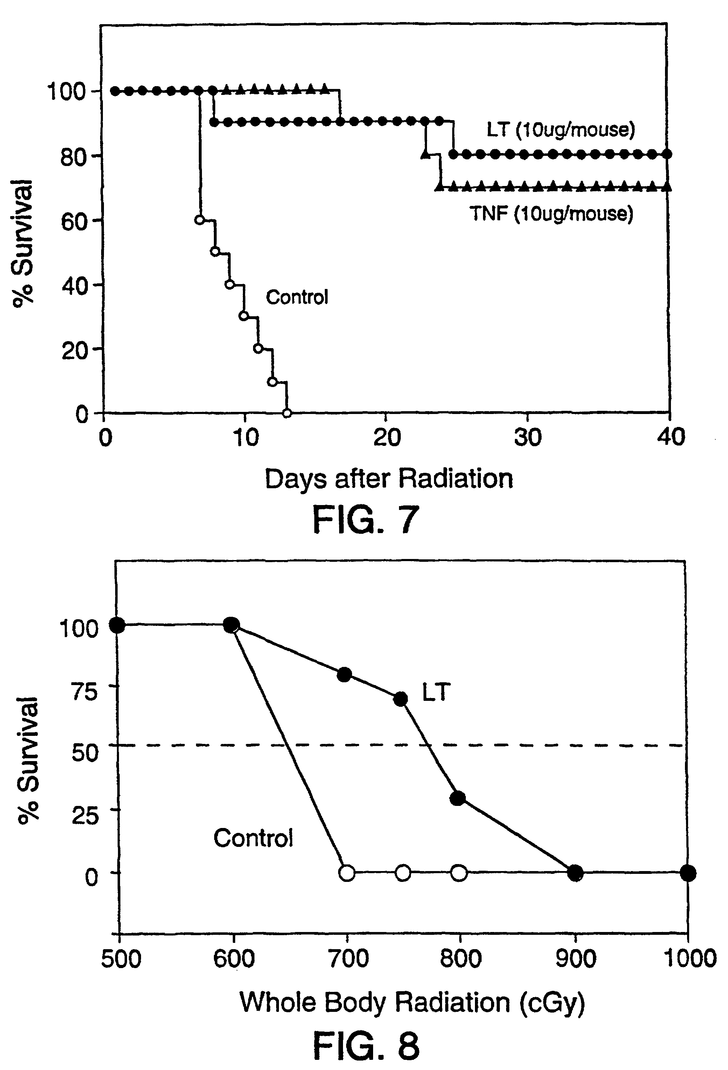

At Day 12 after radiation treatment. 9/10 (90%) of mice pretreated with LT survived whereas all the control tumor bearing mice (10/10) were dead. (See Figure 7). At Day 40, 8/10 mice pretreated with LT were alive and free of tumor cells whereas 7/10 TNF-pretreated mice survived (one mouse having detectable tumor).

A group of the LT treated mice were also radiated with varying doses of radiation. On Day 30 post radiation. it was observed that LT treatment allowed approximately 20% increase in radiation dose to be tolerated at the LD50 level. (Figure 8).

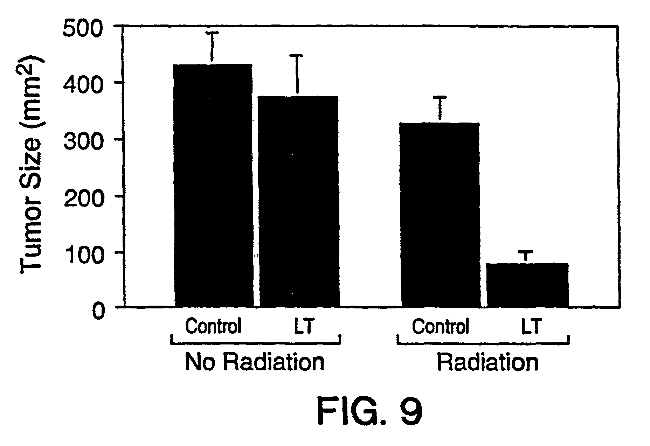

Pretreatment with LT also enhanced sensitivity of the tumor cells to the radiation treatment. Those mice receiving LT prior to radiation (750 cGy) showed significant reduction in tumor size at Day 7. (See Figure 9).

### C. Effect in Radiated Tumor Bearing Nude Mice

The effects of LT and radiation therapy were examined in tumor bearing nude mice (purchased from Harlan Sprague Dawley) using the protocol described in Example 3, Section B above except that HT 1080 human fibrosarcoma cells (obtained from ATCC, No. CCL 121) were injected subcutaneously into the mice.

The LT treated nude mice had 100% survival (10/10 mice) out to 25 days post radiation treatment. In contrast, 50% of the control mice were dead on Day 15 post radiation, and 100% of the control mice were dead on Day 19 post radiation. (data not shown)

Pretreatment with LT also resulted in enhanced degree of necrosis of the tumors on Day 12. as shown in Table 2 below.

**TABLE 2**

| LT PRETREATMENT | RADIATION | DEGREE OF NECROSIS | | | |
|---|---|---|---|---|---|
| | | 0 | 1+ | 2+ | 3+ |
| Control | - | 6 | 4 | | |
| LT | - | 5 | 3 | 2 | |
| Control | + | 5 | 5 | | |
| LT | + | | 3 | 2 | 5 |

### D. Radiosensitization in Nude Mice Bearing Breast Adenocarcinoma Tumor

The effects of LT and radiation therapy in tumor bearing nude mice were examined by treating mice with LT and radiation therapy and then assaying tumor cells *in vitro* in a clonogenic assay.

Nude mice (obtained from Harlan Sprague Dawley) were injected subcutaneously with MDA231 human breast adenocarcinoma cells (obtained from ATCC, No. HTB 26) (10⁶ cells/0.1 ml/mouse) and the tumor cells were allowed to grow for 10 days. LT was injected intraperitoneally (10 µg/0.5ml/mouse) at -2 Days and -1 Day prior to radiation on Day 0. Control animals were similarly treated except that 0.5ml sterile PBS was injected instead of LT. The mice were radiated using 1500 cGy ¹³⁷Cs gamma-ray whole body radiation to mimic local radiation dose in humans. One day after radiation treatment, the mice were sacrificed. The tumors were then isolated and aseptically removed from the subcutaneous site.

Each tumor was placed in a sterile petri dish and 1-2 ml Hank's Balanced Salt Solution ("HBSS") containing antibiotic was added to each dish. The tumors were then chopped into small fragments. The contents of the petri dishes were transferred to 125 ml flasks. Approximately 7-8 ml of HBSS was used to rinse the petri dishes and was then added to the 125 ml flasks containing the tumor fragments.

Single cell suspensions of each tumor were prepared by adding 1 ml enzyme digestion solution to each 125 ml flask. The enzyme digestion solution included HBSS and 6 mg/ml Pronase (purchased from Cal-Biochem), 2 mg/ml DNAse (purchased from Sigma), and 2 mg/ml Collagenase (purchased from Sigma). The enzyme digestion solution and tumor fragments were next incubated for 20 minutes in a warm room on a shaker apparatus. To stop the enzymatic reaction, 7 ml fetal bovine serum was added to each flask. The contents of the flasks were then filtered through sterile gauze to separate out undigested tumor fragments. The filtered cell suspensions were rinsed with cold PBS containing antibiotic and placed in 50 ml conical tubes.

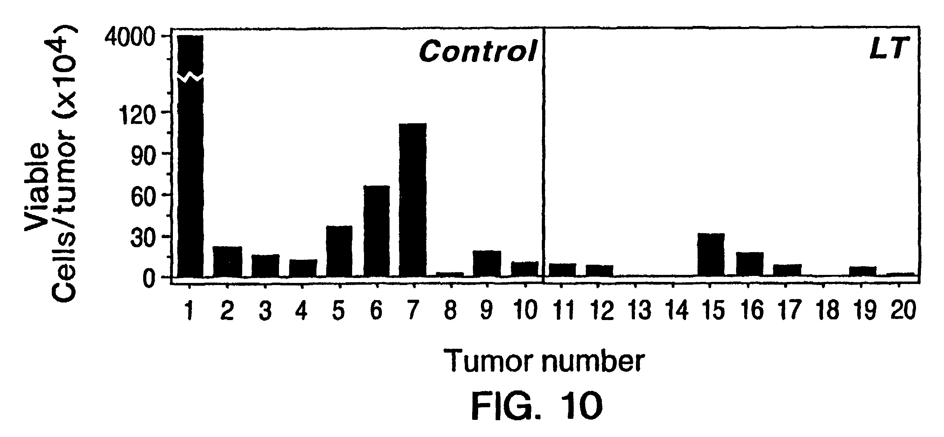

The tumor cells were then tested for cell viability using trypan blue dye exclusion. The LT treated mice showed fewer viable tumor cells remaining after radiation treatment as compared to control animals. (See Figure 10). Three often mice (identified as tumor nos. 13, 14, and 18 on Figure 10) receiving LT had no detectable viable tumor cells.

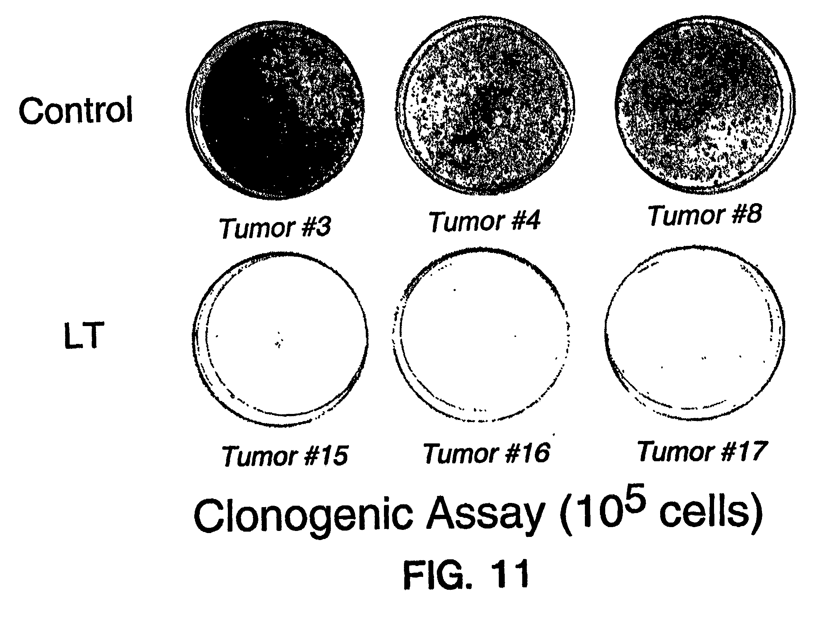

A portion of each single viable cell suspension was also tested in a clonogenic assay. Viable tumor cells from LT treated tumor nos. 15, 16, and 17 (identified in Figure 10) and control tumor nos. 3, 4, and 8 (identified in Figure 10) were plated in high glucose DMEM at a concentration of 10⁵ cells/petri dish and incubated at 37°C. After 2 weeks, cell cultures were stained with 0.5% crystal violet in 20% methanol. As shown in Figure 11, cells from all three control animals continued to grow. In contrast, none of the LT treated tumor cells grew.

### E. Radiosensitization in Nude Mice Bearing Human Primary Tumor

Nude mice were treated essentially as described in Example 3. Section D above, except that the mice were injected with colon cancer cells isolated from a primary tumor in a human patient (designated patient No. 180). The tumor cells were allowed to grow in the nude mice for 14 days. Tumors grew in 12/20 mice injected with the human primary tumor cells.

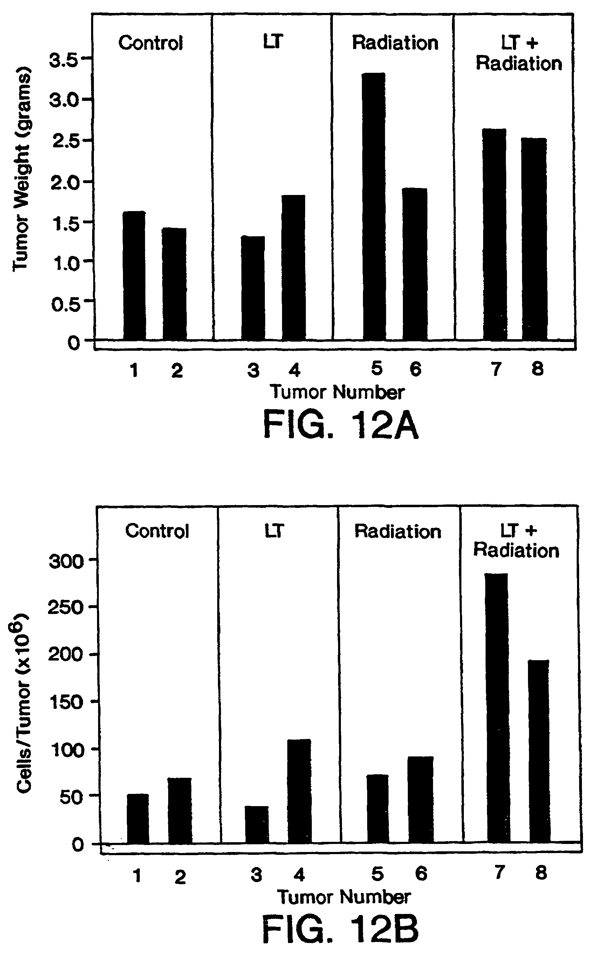

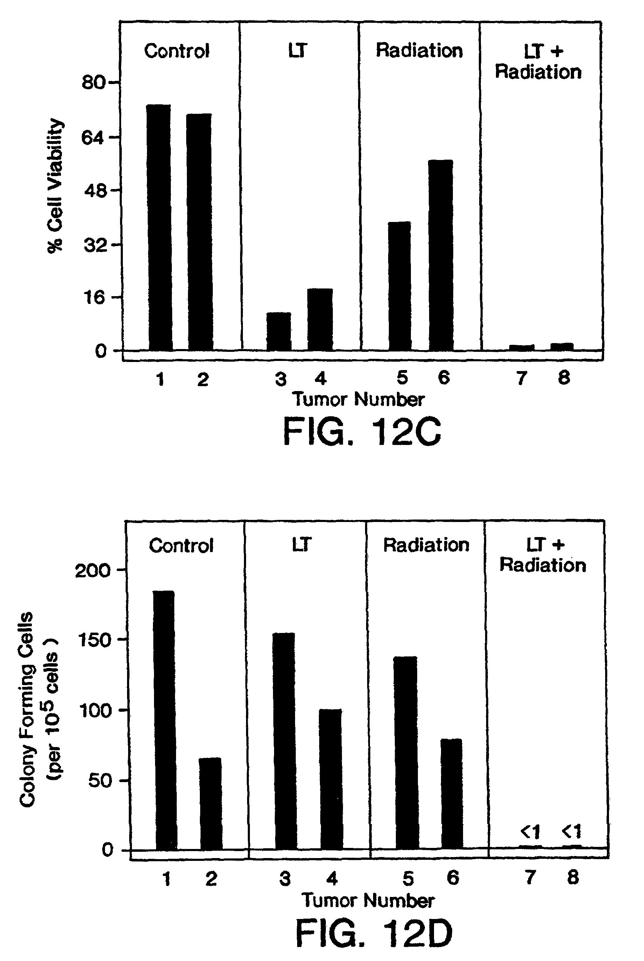

One day after irradiation (1500 cGy) treatment the tumor bearing mice were sacrificed. The tumors were then isolated and aseptically removed from the subcutaneous site. The tumors were first weighed and it was observed that LT treatment did not affect the size (weight) of the intact tumors (Figure 12A). Each tumor was then chopped into fragments and single cell suspensions were prepared as described in Example 3. Section D above. The single cell suspensions were then counted. As shown in Figure 12B. LT and radiation treatment did not result in a decrease in the total cell yield from the tumor. However, when the tumor cell suspensions were tested for cell viability using trypan blue dye exclusion, it was found that the LT-radiation treated mice had tumors with significantly lower cell viability as compared to the control mice. (See Figure 12C).

A portion of each single cell suspension was also tested in a clonogenic assay. Viable tumor cells from the mice were adjusted to a concentration of 10⁶ cells/ml in high glucose DMEM media. Soft agar cultures were prepared by autoclaving a 1% DIFCO Bacto-Agar solution and then incubating the solution at 52°C. The 1% Bacto-Agar solution was mixed with an equal volume of 2x high glucose DMEM media, and 4 ml of the mixture was added to 60 mm petri plates. The agar mixture in the plates was then allowed to solidify. The tumor cells were then plated by adding 100 µl of the cell suspensions (10⁶ cells/ml) to the agar-coated plates. Next, a top layer of agar was added to the petri plates. The top layer of agar was prepared by autoclaving a 0.5% DIFCO Bacto-Agar solution and incubating the solution at 52° C. The 0.5% Bacto-Agar solution was mixed with an equal volume of 2x high glucose DMEM media, and 3 ml of the agar mixture was added to each petri plate. The top layer of agar was then allowed to solidify.

The cultures were incubated at 37° C for two weeks. Next, the tumor cell cultures were stained with 10 mg/ml MTT (3-[4,5 dimethylthiazol-2-yl]-2,5-diphenyltetrazolium bromide) in EtOH. Specifically, 230 µ*l* of the MTT solution was added to 10 ml high glucose DMEM to form a mixture, and I ml of the mixture was added to each petri plate. The petri plates were incubated 12-24 hours at room temperature and the number of colonies in each plate was then counted. As shown in Figure 12D, the tumors from the mice treated with LT and radiation had less than one colony forming cell per 10⁵ cultured tumor cells.

Similar results were obtained in identical experiments testing primary colon tumor cells obtained from another human patient (designated patient No. 179). (data not shown).

### F. Effect on Bone Marrow

Nude mice were treated essentially as described in Example 3, Section A above. Eleven days after radiation on Day 0. the mice were sacrificed. Bone marrow cells were collected by dissecting femur bones and flushing the femoral shafts with sterile PBS. The cells were then washed and resuspended in DMEM medium. Bone marrow cellularity was determined by scoring nucleated cells on a hemocytometer after staining with trypan blue.

For the identification of multipotent hematopoietic progenitor cells, 5 x 10³ bone marrow cells from LT treated mice and control mice were cultured in 1% methylcellulose (4,000 mP. Fluka. A.G., Bachs, Switzerland) in DMEM medium supplemented with 15% fetal bovine serum, 0.45 mM monothioglycerol, 2 mM L-glutamine. 15 mg/ml insulin, 200 mg/ml transferrin, 1,000 U IL-lbeta, 50 U IL-3 (purchased from Genzyme, Cambridge, MA) and either 1 U of GM-CSF or 2.4 U of EPO (IL-1 beta, GM-CSF and EPO were purchased from R & D Systems, Minneapolis, MN). Cells were cultured in a final volume of 1.5 ml in 35 mm petri plates for 10 days, after which time colony formation was determined using a dissecting microscope.

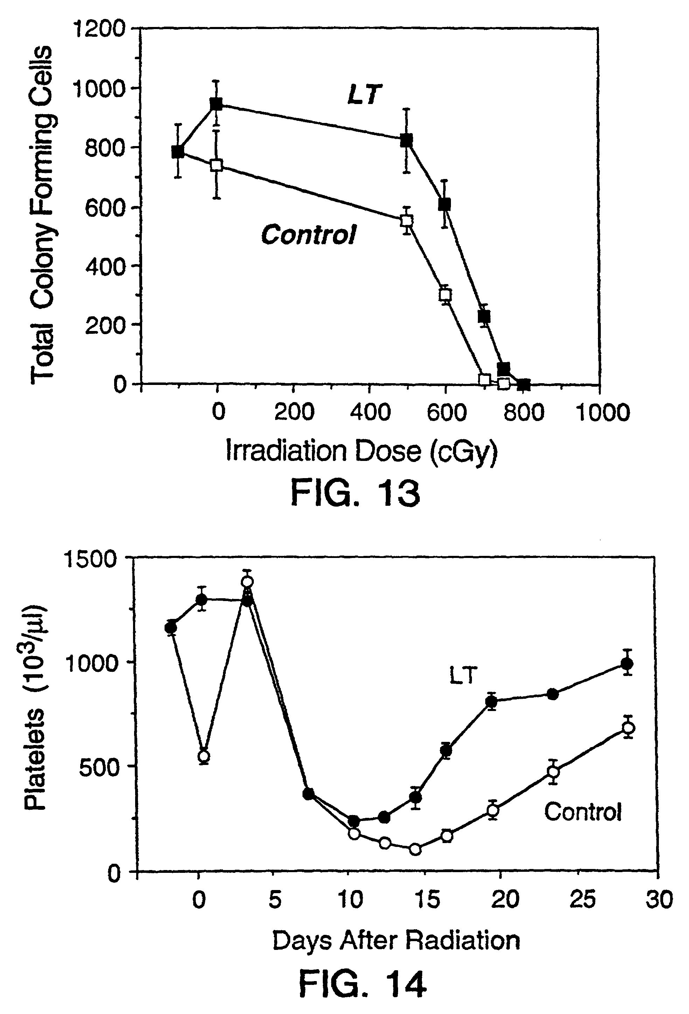

The results are shown in Figure 13. Bone marrow from LT-radiation treated mice gave rise to higher numbers of colony forming cells than the control group, indicating LT may protect progenitor, stem cells or/and bone marrow stromal cells against radiation induced damage.

### G. Effect on Circulating Platelet Levels

C57BL/6 mice (obtained from Harlan Sprague Dawley) were injected intraperitoneally with 10 µg/0.5 ml/mouse LT at -2 Days and -1 Day prior to radiation at Day 0. Control mice were similarly treated except that sterile PBS was injected instead of LT. The mice were then radiated using 750 cGy ¹³⁷Cs gamma-ray whole body radiation.

Blood samples from a randomized number of mice (10) were obtained prior to injecting LT or PBS on -2 Days. These blood samples (40 µl/mouse) were obtained from the orbital sinus of each mouse using micropipettes (Serono Diagnostics. Inc., Allentown, PA) and were immediately diluted in 10 ml of diluent (Haema-Line DIFF Silos, Serono-Baker Diagnostics. Inc., Allentown, PA). The samples were rocked gently to prevent sedimentation of the cells. Within 1 hour of collection, blood samples were analyzed using a Serono Baker System 9000 hematology analyzer. Blood samples were similarly obtained and analyzed on Day 0 (before radiation) and Days 3, 10, 12, 14, 17, 20, 24, and 28 after radiation. Five animals in each group were bled on alternate time points, with the exception of Day 10, on which all the animals were bled. Animals were not bled more than a total of 5 times throughout the study.

As shown in Figure 14, the platelet counts from LT treated mice were significantly higher than the control mice on Days 14, 17, 20, 24 and 28 after radiation. Increased platelet counts were also observed in other strains of mice, such as BALB/c and nude mice, treated with LT prior to lethal radiation. (data not shown).

### Example 4: Effects of LT and

### Radiation Therapy in Vitro

Bone marrow cells from normal BALB/c mice (mice obtained from Harlan Sprague Dawley) and cell lines HL-60 (human promyelocytic leukemia cells obtained from ATCC, No. CCL 240), K-562 (human chronic myelogenous leukemia cells, ATCC No. CCL 243), ME-180 (human epidermoid carcinoma cells, ATCC No. HTB 33), and T-24 (human transitional-cell carcinoma cells, ATCC No. HTB 4) were cultured in RPMI-1640 medium supplemented with 5% fetal calf serum. Test cultures were treated with 1 µg/ml LT. No LT was added to the control cultures. Twelve hours after adding the LT to the test cultures, both test cultures and control cultures were radiated using 1000 cGy ¹³⁷Cs gamma-ray radiation. After 72 hours. the cells were tested for viability by trypan blue dye exclusion. The results of the viability testing are shown in Table 3 below.

**TABLE 3**

| | % VIABILITY | |
|---|---|---|
| CELLS | CONTROL | LT TREATMENT |
| Normal BALB/c Bone Marrow | 28 +/- 7 | 65 +/- 6 |
| HL-60 | 25 +/- 3 | <1 |
| K-562 | 31 +/- 5 | <1 |
| ME-180 | 22 +/- 2 | <1 |
| T-24 | 47 +/- 11 | <1 |

The cancer cell lines treated with LT prior to radiation had <1% viability 72 hours after radiation treatment The normal BALB/c bone marrow cells treated with LT prior to radiation, in contrast, showed enhanced viability after radiation.

### Example 5: Effects of LT and Thrombopoietin on Circulating Platelet Levels in Sublethally Radiated Mice

In a first study, four groups of C57BL/6 mice (obtained from Harlan Sprague Dawley) were treated as outlined in Table 4 below.

**Table 4**

| **Group** | **Treatment/Mouse** | | |
|---|---|---|---|
| | Days -2, -I | Day 0 | Days 1-16 |
| Buffer Control | 0.5*ml* | radiation | 100*µl* |
| | PBS | 750cGy | PBS-0.01% |
| | | | Tween-80 |
| TPO332 | 0.5*ml* | radiation | 0.3µ*g*/100µ*l* |
| | PBS | 750cGy | TP0332 |
| LT | 10µ*g*/0.5*ml* | radiation | 100µ*l* |
| | LT | 750cGy | PBS-0.01% |
| | | | Tween-80 |
| LT/TPO332 | 10µ*g*/0.5*ml* | radiation | 0.3µ*g*/100µ*l* |
| | LT | 750cGy | TP0332 |

The LT and buffer controls were injected intraperitoneally. The purified TPO332 (diluted in PBS-0.01% Tween-80) was injected subcutaneously. The respective injections were administered on each of Days -2 and -1, and on Days 1 through 16 of the study. On Day O, all the animals were radiated with 750 cGy of ¹³⁷Cs gamma-ray whole body sublethal radiation.

Blood samples from a randomized number of mice were obtained prior to injecting LT or buffer on Day -2. These blood samples (40 µl/mouse) were obtained from the orbital sinus of each mouse using micropipettes (Serono Diagnostics, Inc., Allentown, PA) and were immediately diluted in 10 ml of diluent (Haema-Line DIFF Silos, Serono-Baker Diagnostics, Inc., Allentown, PA). The samples were rocked gently to prevent sedimentation of the cells. Within 1 hour of collection, blood samples were analyzed using a Serono Baker System 9000 hematology analyzer. Blood samples were similarly obtained from a random sample of the experimental animals and analyzed on Day 0 (before radiation). On Days 3, 7, 10, 12, 14, 17, 21, 23, and 28 after radiation, five animals in each group were bled. Animals were bled on alternate time points. with the exception of Days 10 and 28, on which all the animals were bled. Animals were not bled more than a total of 8 times throughout the study.

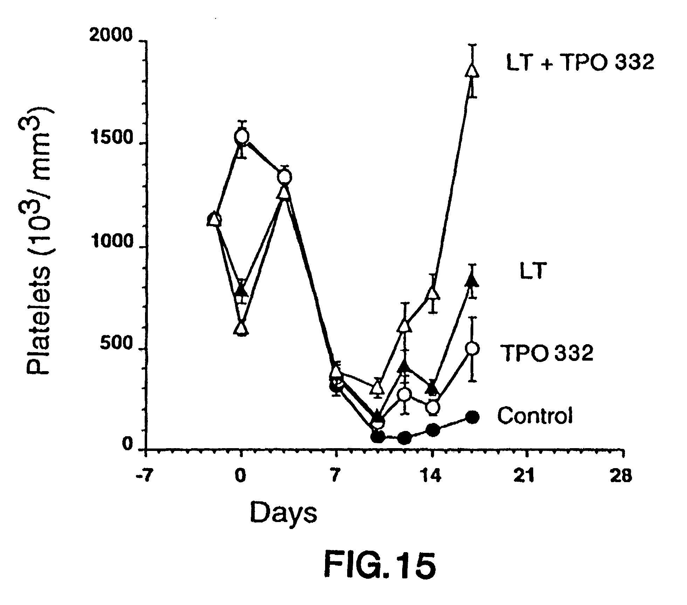

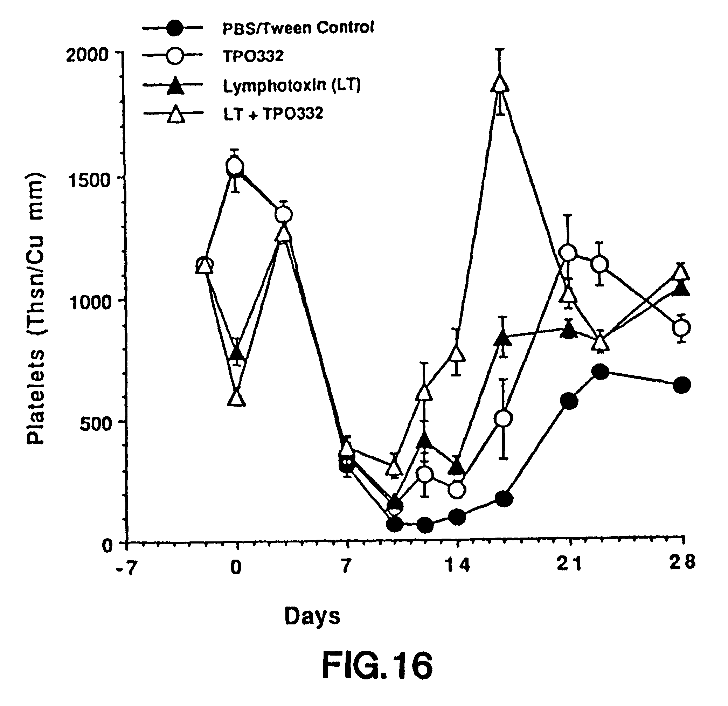

As shown in Figure 15, the platelet counts (from samples collected at Day 14 and Day 17) from the animals treated with both LT and TPO332 were significantly higher as compared to animals treated with LT or TPO332 alone. Overall, LT/TPO332 treatment dramatically enhanced the recovery of platelet levels following sublethal radiation. Platelet levels determined in Days 21-28 of the study showed a return to normal ranges. (See Figure 16). Although not wishing to be bound by any particular theory, it is presently believed that the LT may protect megakaryocyte or bone marrow progenitor cells, as well as bone marrow stromal cells, against radiation induced damage. Alternatively, it is believed that LT may induce the synthesis of thrombopoietin or other growth factors that synergize thrombopoietin function.

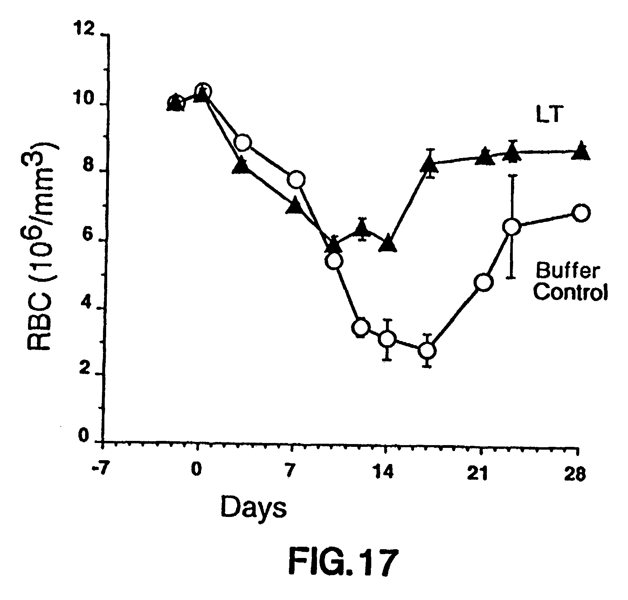

The blood samples were also analyzed for RBC levels. Recovery of RBC levels in the animals treated with LT alone was significantly enhanced at Days 10, 12, and 14 as compared to the buffer control treated animals. (See Figure 17).

In a second study, C57BL/6 mice (obtained from Harlan Sprague Dawley) were treated as outlined in Table 5 below.

**Table 5**

| **Group** | **Treatment/Mouse** | | |
|---|---|---|---|
| | Days-2,-1 | Day0 | Days 1-14 |
| Buffer Control | 0.5*ml* | radiation | 100*µl* |
| | PBS | 750cGy | 10mM Tris, |
| | | | 140mM NaCl. |
| | | | 0.01% Tween-20 |
| | | | pH 7.4 |
| TP0332 | 0.5*ml* | radiation | 1.0µg/100µ*l* |
| | PBS | 750cGy | or |
| | | | 0.3µg/100µ*l* |
| | | | TPO332 |
| LT/TPO332 | 0-10µg/0.5*ml* LT | radiation | 1.0µg/100*ml* |
| | | 750cGy | or |
| | | | 0.3*µl*/100*µl* |
| | | | TP0332 |

Within the groups receiving either TPO332 or both LT and TPO332, the animals were divided into further groups to receive varying doses of TPO332 and LT. TPO332 was administered at doses of either 0.3 µg or 1.0 µg. LT was administered at doses of 10 µg, 5 µg, 2 µg, 0.5 µg or 0 µg. The LT and buffer controls were injected intraperitoneally. The purified TPO332 (diluted in 10mM Tris, 140mM NaCI, 0.01% Tween-20, pH 7.4) was injected subcutaneously. The respective injections were administered on each of Days -2 and - I, and on Days I through 14 of the study. On Day O, all the animals were radiated with 750 cGy of ¹³⁷Cs gamma-ray whole body radiation.

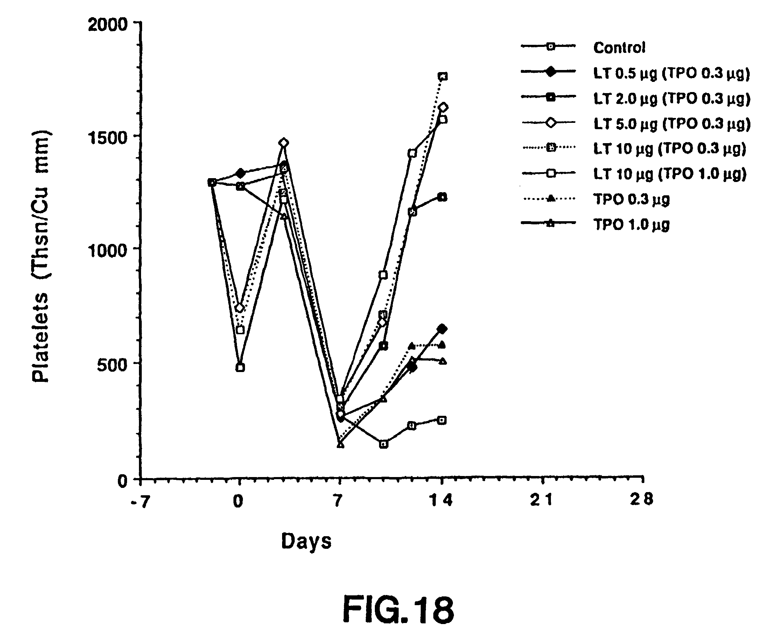

Blood samples were obtained prior to injecting LT or buffer on Day -2, as described above. Blood samples were similarly obtained from half of the animals in each LT treatment group and analyzed on Day 0 (before radiation) and Days 3, 7, 10, 12, and 14 after radiation, as described above. The results are illustrated in the graph of Figure 18. As shown in Figure 18, LT treatment of 2, 5, and 10 µg markedly enhanced the recovery of platelets at Days 10, 12 and 14 of the study, whereas *0.5* µg showed no significant enhancement. Thus, it is believed that the activity of LT is dose dependent.

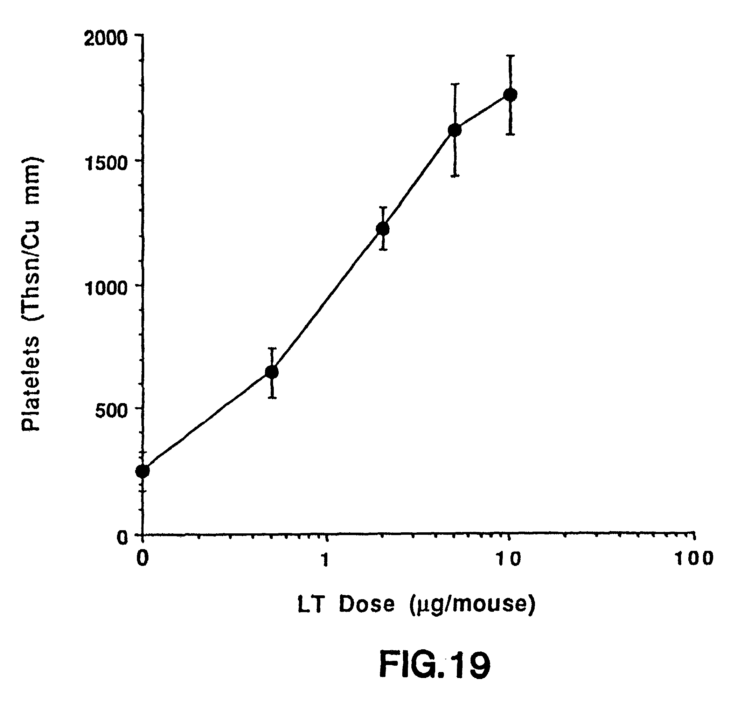

Figure 19 shows the dose response curve for LT (injected on Days -2 and - 1) administered in combination with 0.3 µg TPO332 (injected on Days 1-14), and as measured from blood samples obtained on Day 14 of the study. The graph illustrates that the ability of LT, with TPO332, to enhance platelet counts is dose dependent with optimal doses of LT at about 5 µg/animal to about 10 µg/animal. This *dose* is consistent with the dose observed for LT in protecting mice against a lethal dose of radiation and sensitizing tumor cells to radiation killing.

The animals in the second study were sacrificed on Day 18 and tissues were analyzed for pathology. None of the tissues showed any observable toxic effects from the TPO332 or LT/TPO332 treatment

## Claims

1. Use of lymphotoxin and thrombopoietin in the preparation of a medicament for enhancing stimulation of megakaryocyte maturation or thrombocyte production in a mammal.

2. The use of claim 1 wherein said lymphotoxin and thrombopoietin are to be administered to the mammal in separate pharmaceutically-acceptable carriers.

3. The use of claim 1 or claim 2 wherein said lymphotoxin and thrombopoietin are to be administered to the mammal sequentially.

4. The use of claim 3 wherein said lymphotoxin is to be administered to the mammal prior to administering the thrombopoietin.

5. The use of claim 4 wherein at least two doses of said lymphotoxin is to be administered to the mammal prior to administering the thrombopoietin.

6. The use of claim 5 wherein multiple doses of thrombopoietin are to be administered to the mammal.

7. The use of any one of the preceding claims wherein said lymphotoxin and thrombopoietin are to be administered to a mammal receiving chemotherapy or radiation therapy.

8. The use of claim 7 wherein said mammal has cancer.

9. The use of any one of the preceding claims wherein said lymphotoxin is recombinant human lymphotoxin and said thrombopoietin is recombinant human thrombopoietin.

10. The use of claim 9 wherein said lymphotoxin is histidyl amino-terminal lymphotoxin and said thrombopoietin is full length c-Mpl ligand.

11. Use of lymphotoxin and thrombopoietin in the preparation of a medicament for administration to a mammal in combination with chemotherapy or radiation therapy.

12. The use of claim 11 wherein said lymphotoxin is to be administered prior to administering said chemotherapy and said thrombopoietin is administered after said chemotherapy.

13. The method of claim 11 wherein said lymphotoxin is to be administered prior to administering said radiation therapy and said thrombopoietin is administered after said radiation therapy.

14. Use of lymphotoxin and thrombopoietin in the preparation of a medicament for the treatment of thrombocytopenia in a mammal.

15. A method for enhancing stimulation of megakaryocyte maturation or thrombocyte production *in vitro,* comprising culturing a cell sample suspected of containing megakaryoblasts, promegakaryocytes and/or basophilic megakaryocytes in the presence of effective amounts of lymphotoxin and thrombopoietin.

16. The method of claim 15 wherein said cell sample is bone marrow.

17. The method of claim 16 wherein said bone marrow is human bone marrow which is cultured in the presence of effective amounts of recombinant human lymphotoxin and recombinant human thrombopoietin.

18. A composition comprising lymphotoxin and thrombopoietin for use in a method of medical treatment.

19. A composition comprising lymphotoxin and thrombopoietin, said lymphotoxin and thrombopoietin being present in amounts effective for enhancing stimulation of megakaryocyte maturation or thrombocyte production.

20. The composition of claim 19 further comprising at least one pharmaceutically-acceptable carrier.

21. The composition of claim 19 wherein said lymphotoxin is recombinant human lymphotoxin and said thrombopoietin is recombinant human thrombopoietin.

22. The composition of claim 21 wherein said lymphotoxin is histidyl amino-terminal lymphotoxin and said thrombopoietin is full length c-Mpl ligand.

## Patentansprüche

1. Verwendung von Lymphotoxin und Thrombopoietin zur Herstellung eines Medikaments zur Verstärkung der Stimulation von Megakaryozyten-Reifung oder Thrombozyten-Produktion bei einem Säugetier.

2. Verwendung nach Anspruch 1, worin das Lymphotoxin und das Thrombopoietin dem Säugetier in getrennten pharmazeutisch annehmbaren Trägern zu verabreichen sind.

3. Verwendung nach Anspruch 1 oder 2, worin das Lymphotoxin und das Thrombopoietin dem Säugetier nacheinander zu verabreichen sind.

4. Verwendung nach Anspruch 3, worin das Lymphotoxin dem Säugetier vor der Verabreichung des Thrombopoietins zu verabreichen ist.

5. Verwendung nach Anspruch 4, worin dem Säugetier vor der Verabreichung des Thrombopoietins zumindest zwei Dosen des Lymphotoxins zu verabreichen sind.

6. Verwendung nach Anspruch 5, worin dem Säugetier mehrere Dosen Thrombopoietin zu verabreichen sind.

7. Verwendung nach einem der vorangegangenen Ansprüche, worin das Lymphotoxin und das Thrombopoietin einem Säugetier zu verabreichen sind, das Chemotherapie oder Strahlentherapie erhält.

8. Verwendung nach Anspruch 7, worin das Säugetier Krebs hat.

9. Verwendung nach einem der vorangegangenen Ansprüche, worin das Lymphotoxin rekombinantes Human-Lymphotoxin ist und das Thrombopoietin rekombinantes Human-Thrombopoietin ist.

10. Verwendung nach Anspruch 9, worin das Lymphotoxin Lymphotoxin mit Histidyl am Aminoterminus ist und das Thrombopoietin c-Mpl-Ligand voller Länge ist.

11. Verwendung von Lymphotoxin und Thrombopoietin zur Herstellung eines Medikaments zur Verabreichung an ein Säugetier in Kombination mit Chemotherapie oder Strahlentherapie.

12. Verwendung nach Anspruch 11, worin das Lymphotoxin vor der Verabreichung der Chemotherapie zu verabreichen ist und das Thrombopoietin nach der Chemotherapie verabreicht wird.

13. Verfahren nach Anspruch 11, worin das Lymphotoxin vor der Verabreichung der Strahlentherapie zu verabreichen ist und das Thrombopoietin nach der Strahlentherapie verabreicht wird.

14. Verwendung von Lymphotoxin und Thrombopoietin zur Herstellung eines Medikaments zur Behandlung von Thrombopenie bei einem Säugetier.

15. Verfahren zum Verstärken der Stimulation von Megakaryozyten-Reifung oder der Thrombozyten-Produktion in vitro, umfassend das Kultivieren einer Zellprobe, von der vermutet wird, dass die Megakaryoblasten, Promegakaryozyten und/oder basophile Megakaryozyten enthält, in Gegenwart wirksamer Mengen an Lymphotoxin und Thrombopoietin.

16. Verfahren nach Anspruch 15, worin die Zellprobe Knochenmark ist.

17. Verfahren nach Anspruch 16, worin das Knochenmark menschliches Knochenmark ist, das in Gegenwart wirksamer Mengen an rekombinantem Human-Lymphotoxin und rekombinantem Human-Thrombopoietin kultiviert wird.

18. Lymphotoxin und Thrombopoietin umfassende Zusammensetzung zur Verwendung in einem medizinischen Behandlungsverfahren.

19. Zusammensetzung, die Lymphotoxin und Thrombopoietin umfasst, wobei das Lymphotoxin und das Thrombopoietin in Mengen enthalten sind, welche die Stimulation von Megakaryozyten-Reifung oder Thrombozyten-Produktion wirksam verstärken.

20. Zusammensetzung nach Anspruch 19, die weiters zumindest einen pharmazeutisch annehmbaren Träger umfasst.

21. Zusammensetzung nach Anspruch 19, worin das Lymphotoxin rekombinantes Human-Lymphotoxin ist und das Thrombopoietin rekombinantes Human-Thrombopoietin ist.

22. Zusammensetzung nach Anspruch 21, worin das Lymphotoxin Lymphotoxin mit Histidyl am Aminoterminus ist und das Thrombopoietin c-Mpl-Ligand voller Länge ist.

## Revendications

1. Utilisation d'une lymphotoxine et de thrombopoïétine dans la préparation d'un médicament destiné à accroître la stimulation de la maturation des mégacaryocytes ou de la production de thrombocytes chez un mammifère.

2. Utilisation suivant la revendication 1, dans laquelle la lymphotoxine et la thrombopoïétine sont destinées à être administrées au mammifère dans des supports pharmaceutiquement acceptables distincts.

3. Utilisation suivant la revendication 1 ou la revendication 2, dans laquelle la lymphotoxine et la thrombopoïétine sont destinées à être administrées successivement au mammifère.

4. Utilisation suivant la revendication 3, dans laquelle la lymphotoxine est destinée à être administrée au mammifère avant l'administration de la thrombopoïétine.

5. Utilisation suivant la revendication 4, dans laquelle au moins deux doses de la lymphotoxine sont destinées à être administrées au mammifère avant l'administration de la thrombopoïétine.

6. Utilisation suivant la revendication 5, dans laquelle des doses multiples de thrombopoïétine sont destinées à être administrées au mammifère.

7. Utilisation suivant l'une quelconque des revendications précédentes, dans laquelle la lymphotoxine et la thrombopoïétine sont destinées à être administrées à un mammifère soumis à une chimiothérapie ou radiothérapie.

8. Utilisation suivant la revendication 7, dans laquelle le mammifère souffre d'un cancer.

9. Utilisation suivant l'une quelconque des revendications précédentes, dans laquelle la lymphotoxine consiste en lymphotoxine humaine recombinante et la thrombopoïétine consiste en thrombopoïétine humaine recombinante.

10. Utilisation suivant la revendication 9, dans laquelle la lymphotoxine est une lymphotoxine à fonction histidyle amino-terminale et la thrombopoïétine consiste en le ligand c-Mpl total.

11. Utilisation d'une lymphotoxine et de thrombopoïétine dans la préparation d'un médicament destiné à l'administration à un mammifère, en association avec une chimiothérapie ou radiothérapie.

12. Utilisation suivant la revendication 11, dans laquelle la lymphotoxine est destinée à être administrée avant l'administration de la chimiothérapie et la thrombopoïétine est administrée après ladite chimiothérapie.

13. Méthode suivant la revendication 11, dans laquelle la lymphotoxine est destinée à être administrée avant l'administration de la radiothérapie et la thrombopoïétine est administrée après ladite radiothérapie.

14. Utilisation d'une lymphotoxine et de thrombopoïétine dans la préparation d'un médicament destiné au traitement d'une thrombocytopénie chez un mammifère.

15. Méthode pour accroître la stimulation de la maturation des mégacaryocytes ou de la production de thrombocytes *in vitro*, comprenant la culture d'un échantillon de cellules supposé contenir des mégacaryoblastes, des promégacaryocytes et/ou des mégacaryocytes basophiles en présence de quantités efficaces d'une lymphotoxine et d'une thrombopoïétine.

16. Méthode suivant la revendication 15, dans laquelle l'échantillon de cellules consiste en moelle osseuse.

17. Méthode suivant la revendication 16, dans laquelle la moelle osseuse consiste en moelle osseuse humaine qui est cultivée en présence de quantités efficaces d'une lymphotoxine humaine recombinante et d'une thrombopoïétine humaine recombinante.

18. Composition comprenant une lymphotoxine et une thrombopoïétine, destinée à être utilisée dans une méthode de traitement médicale.

19. Composition comprenant une lymphotoxine et une thrombopoïétine, ladite lymphotoxine et ladite thrombopoïétine étant présentes en des quantités efficaces pour accroître la stimulation de la maturation des mégacaryocytes ou de la production de thrombocytes.

20. Composition suivant la revendication 19, comprenant en outre au moins un support pharmaceutiquement acceptable.

21. Composition suivant la revendication 19, dans laquelle la lymphotoxine consiste en lymphotoxine humaine recombinante et la thrombopoïétine consiste en thrombopoïétine humaine recombinante.

22. Composition suivant la revendication 21, dans laquelle la lymphotoxine est une lymphotoxine à fonction histidyle amino-terminale et la thrombopoïétine consiste en le ligand c-Mpl total.
